# FASCICULE DE BREVET EUROPEEN

(11) **EP 2 688 880 B1**
(45) Date de publication et mention de la délivrance du brevet: **12.12.2018**
(21) Numéro de dépôt: 12717367.2
(22) Date de dépôt: 23.03.2012
(51) Int. Cl.: C07D 401/14

(54) **COMPLEXES MÉTALLIQUES DINUCLEAIRES GREFFES, ET LEUR UTILISATION EN TANT QUE CAPTEURS DE MICROPARTICULES CELLULAIRES**
DINUKLEARE GEPFROPFTE METALLKOMPLEXE UND IHRE VERWENDUNG ALS ZELLULÄRE MIKROPARTIKELSENSOREN
GRAFTED DINUCLEAR METAL COMPLEXES, AND USE THEREOF AS CELLULAR MICROPARTICLE SENSORS

(30) Priorité: 23.03.2011 FR 1100873
(43) Date de publication de la demande: 29.01.2014
(73) Titulaire: Centre National de la Recherche Scientifique (C.N.R.S.), 75016 Paris (FR); Université de Caen Basse Normandie, 14000 Caen (FR); Université Joseph Fourier, 38041 Grenoble Cedex 9 (FR)
(72) Inventeur: BELLE, Catherine, 38470 Beaulieu (FR); GELLON, Gisèle, 38400 Saint-martin d'Heres (FR); PLAWINSKI, Laurent, 14310 Epinay sur Odon (FR); DOEUVRE, Loïc, 14530 Luc sur Mer (FR); ANGLES CANO, Eduardo, 750270 Paris Cedex 06 (FR)
(74) Mandataire: Santarelli
(86) Numéro de dépôt international: PCT/FR2012/050610
(87) Numéro de publication internationale: WO 2012/127175

(56) Documents cités:
- INOMATA, TOMOHIKO ET AL: "Self-assembled monolayer electrode of a diiron complex with a phenoxo-based dinucleating ligand: observation of molecular oxygen adsorption/desorption in aqueous media", CHEMICAL COMMUNICATIONS (CAMBRIDGE, UNITED KINGDOM) , (3), 392-394 CODEN: CHCOFS; ISSN: 1359-7345, 2008, XP002640577,
- KWON, TAE-HYUK ET AL: "Phosphorescent thymidine triphosphate sensor based on a donor-acceptor ensemble system using intermolecular energy transfer", CHEMISTRY--A EUROPEAN JOURNAL , 14(31), 9613-9619 CODEN: CEUJED; ISSN: 0947-6539, 2008, XP002640578,

## Description

L'invention concerne de nouveaux complexes métalliques dinucléaires greffés et leur utilisation en tant que capteurs dans une méthode de détection et/ou de caractérisation de microparticules cellulaires en milieu supporté (ou hétérogène) ou en solution.

Les termes « microparticule cellulaire» ou « microvésicule cellulaire » peuvent être utilisés indifféremment. Par commodité, on utilisera dans la suite de la description le terme « microparticule » ou « microparticule cellulaire ».

Les microparticules cellulaires sont des microvésicules membranaires libérées dans les fluides biologiques lors de l'activation des cellules, ou durant l'apoptose, au cours d'états pathologiques variés, parmi lesquels l'inflammation ou des maladies associées à l'altération de la fonction vasculaire.

Ces microparticules sont libérées dans l'espace extracellulaire suite à un remodelage de la membrane cellulaire, exposant ainsi sur le feuillet externe la phosphatidylsérine, normalement présente sur le feuillet interne, ainsi que d'autres marqueurs identitaires de leur origine cellulaire.

Les microparticules cellulaires ont une taille inférieure au micron (0,1-1 µm). Bien qu'elles soient présentes dans les fluides biologiques corporels, notamment dans le sang circulant, de sujets sains, leur présence en taux élevé est associée à diverses pathologies thrombotiques, inflammatoires ou métaboliques et dans le cancer. Outre le cancer, ces pathologies sont nombreuses et vont du diabète et ses complications vasculaires aux maladies inflammatoires, incluant l'athérosclérose. Il s'agit, en particulier, des maladies cardiovasculaires à risque thrombotique accru ou encore des accidents ischémiques neurovasculaires.

Les microparticules cellulaires peuvent donc être considérées des marqueurs précoces permettant de diagnostiquer et d'évaluer le risque de développement de diverses pathologies, notamment thrombotiques, inflammatoires et/ou métaboliques, et d'en permettre le suivi thérapeutique.

Les accidents neurovasculaires et les maladies occlusives cardiovasculaires constituent, juste après le cancer, des causes majeures de morbidité et de mortalité. Ces pathologies entrainent, en Europe et aux Etats-Unis, des coûts importants en matière sociale et économique et en termes de santé publique.

Ces accidents occlusifs sont le résultat d'un thrombus formé *in situ* ou d'une embolie. La formation d'un thrombus occlusif a été associée à un défaut de fibrinolyse localisée. Cependant, malgré ce contexte physiopathologique, on ne dispose pas actuellement de méthodologie fiable pour évaluer l'activité fibrinolytique dans l'espace intravasculaire permettant l'évaluation des risques et la prévention.

Plusieurs études ont montré que des concentrations élevées de microparticules cellulaires avaient une bonne valeur pronostique.

Des méthodes de détection ont été établies. Parmi les quelques techniques disponibles figure la cytométrie de flux, qui est la plus largement utilisée. Cette méthode permet partiellement le phénotypage et la quantification partielle des microparticules cellulaires à l'aide de billes de calibration. Cette méthode met à profit l'affinité de la phophatidylsérine avec l'annexine A5, une protéine cellulaire. Plus généralement, on utilise des anticorps dirigés contre des déterminants antigéniques exposés sur leurs membranes.

Les microparticules cellulaires peuvent notamment être détectées par mesure de la fluorescence émise par l'annexine A5 ou par des anticorps marqués par un composé fluorescent.

Cependant la sensibilité de cette méthode ne permet pas l'identification de microparticules cellulaires de taille inférieure à 500nm.

L'annexine A5 est également utilisée dans un test de "capture/quantification" dans lequel les microparticules cellulaires qui exposent la phosphatidylsérine sont capturées par l'intermédiaire de l'annexine A5 immobilisée à la surface de puits de plaques à microtitration (test développé par la société Hyphen Biomed).

Cependant cette technique ne permet pas d'obtenir le phénotype et la répartition des éléments. En outre, sa mise en oeuvre est lourde et dépend de nombreux facteurs expérimentaux. La capture des microparticules cellulaires par l'annexine A5 est fortement dépendante des ions calciques et est très sensible aux variations expérimentales (protéolyse, interactions avec divers ligands, etc.).

L'article Kwon et al., Chem. Eur. J., 2008, 14, 9613-9619 décrit la détection sélective d'un nucléotide particulier, la thymidine 5'-triphosphate (TTP).

Cette détection est effectuée par un système donneur / accepteur dans lequel on détecte une fluorescence accrue due à la liaison de l'unité thymine du TTP avec, d'une part, un complexe de zinc (mCP-Zn²⁺ -cyclen) servant de donneur et, d'autre part, un composé trinucléaire 2 comprenant un motif complexe d'iridium (motif accepteur) et un motif complexe de zinc.

Ainsi, les techniques de détection disponibles manquent de précision et de sensibilité. De plus, elles sont souvent très coûteuses.

Il est donc recherché de pouvoir détecter, quantifier et caractériser, notamment phénotyper, de manière précise et standardisée les microparticules cellulaires dans les fluides biologiques.

Par « fluide biologique » on entend tout liquide corporel extractible dont, par exemple, le sang, le plasma sanguin, le liquide céphalorachidien, le liquide broncho-alvéolaire, l'urine, le liquide synovial, le lait maternel, la salive, les larmes, le liquide séminal, les liquides d'ascite, le liquide amniotique et les épanchements (pleural ou autres).

Les inventeurs ont à présent mis au point une méthode de détection et/ou de caractérisation dans laquelle les microparticules cellulaires sont captées par un ligand synthétique.

La méthode selon l'invention permet la détection et/ou la caractérisation des microparticules cellulaires quelle que soit leur taille. En outre, l'utilisation d'un ligand synthétique permet d'éviter les inconvénients liés à l'utilisation d'une molécule biologique, tels que, par exemple, l'interférence avec d'autres ligands, notamment des anticorps, la protéolyse ou encore la sensibilité au calcium.

Selon un premier aspect, l'invention concerne donc des complexes métalliques dinucléaires greffés susceptibles de reconnaître et de fixer spécifiquement la phosphatidylsérine externalisée à la surface des microparticules.

Dans la suite de la description, on utilisera indifféremment l'expression « complexes métalliques dinucléaires greffés » ou « capteurs » pour définir les composés de formule (I) ou (II) ci-dessous.

L'invention a donc pour objet un composé de formule (I) ou de formule (II) suivante : 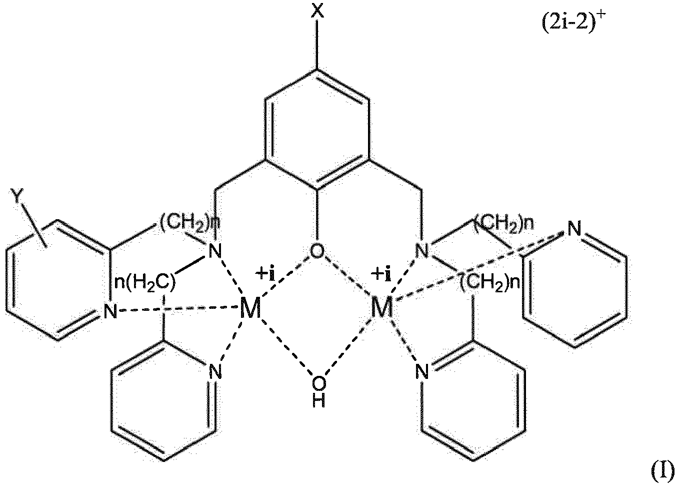 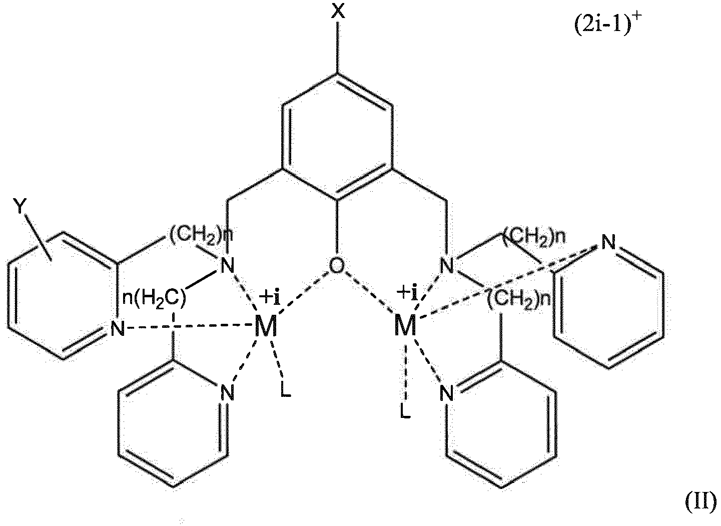 dans laquelle
- M⁺ⁱ représente un ion métallique et i est 1, 2 ou 3
- L représente un ligand échangeable
- X représente un groupe -(CH₂)ₘ-NH-A, ou un groupe -CH₂-NHC(O)-R-NH-A dans lequel R est un groupe alkyle en C₂-C₁₀, de préférence en C₆-C₁₀, linéaire ou ramifié et A est H ou un groupe fluorophore
- m = 1 à 12
- n = 1, 2 ou 3
- i représente la charge du métal et est 1, 2 ou 3
- Y représente (CH₂)ₚ-NH₂ où p = 0 à 12.

De préférence, M est choisi parmi Zn, Cu, Mn, Co, Ni et Fe, Zn ou Cu étant préférés.

En solution aqueuse, les composés de formule (I) et (II) peuvent être en équilibre, en fonction du pH du milieu. Par exemple pour X=CH₃, n=1 et M = Cu ou Zn, on observe qu'à pH physiologique, les deux espèces sont présentes.

Ce sont des composés cationiques dont le contre-ion peut être choisi, par exemple, parmi les anions tosylate, nitrate, sulfate, sulfonate, thiosulfate, halogénure, hexafluorophosphate, tétraphénylborate, tétrafluoroborate etc.

Par « ligand échangeable », on entend une molécule qui interagit de manière faible avec les ions métalliques et qui est disponible pour l'échange avec la solution environnante, telle que par exemple une molécule de solvant, en particulier une molécule de H₂O ou d'un autre solvant tel que, par exemple l'acétonitrile, ou encore une molécule dissoute dans le solvant.

En effet, les molécules présentes dans la solution peuvent avoir une affinité pour un ion métallique donné, ce qui se traduit par des effets compétitifs au niveau des ligands échangeables.

Dans la suite de la description, on entendra par « ligand » la partie dinucléaire du composé de formule (I) ou (II) avant complexation par l'ion métallique, et par « complexe métallique dinucléaire » ou « capteur» le ligand complexé.

Les composés de formule (I) ou (II) peuvent être préparés en adaptant les procédés décrits dans C. Belle et al., Tetrahedron Letters, 1994, 35, 7019-7022, pour la partie ligand, et dans K. Selmeczi et al., Chem. Eur. J., 2007, 13, 9093-9106, pour ce qui concerne la complexation. L'isolement du complexe final est adapté en fonction de la forme du composé obtenu, par des techniques usuelles (précipitation éventuelle, récupération d'une poudre, d'une huile. etc.)

Le procédé de préparation des composés de formule (I) ou (II) peut notamment comprendre les étapes suivantes :
a) synthèse d'un précurseur de ligand dont la fonction amine, lorsque A = H, est protégée par un groupement protecteur à partir de la bis(2-pyridylméthyl)amine ou BPA et de la 2₋[3,5-Bis(chlorométhyl)-4-hydroxybenzyl]isoindole-1,3-di-one,
b) déprotection du ligand ainsi obtenu, puis
c) complexation de ce dernier par l'ion métallique.

Comme groupement protecteur de la fonction amine, on utilisera, par exemple, un groupement phtalimide, tert-butyloxycarbonyl (BOC), 9-fluorenylmethyloxycarbonyl (FMOC), acétyl, tosyl ou carbamate.

La BPA est disponible commercialement et la 2-[3,5-Bis(chlorométhyl)-4-hydroxybenzyl]isoindole-1,3-di-one est décrite dans la publication M. Johansson et al., Inorg. Chem.2003, 42, 7502-7511.

La synthèse d'un précurseur de ligand dont la fonction amine est protégée par un groupement phtalimide à partir de la BPA et de la 2-[3,5-Bis(chlorométhyl)-4-hydroxybenzyl]isoindole-1,3-di-one peut être, par exemple, effectuée comme indiqué dans les exemples 1 et 2.

Le groupement X et/ou le groupement Y, s'il(s) existe(ent), également appelé(s) « bras », peut (peuvent) être greffé(s) sur l'unité phénol du ligand avant complexation de l'ion métallique, soit, dans le schéma 1, en partant du composé 4.

Selon un aspect de l'invention, le bras greffé sur l'unité phénol du complexe métallique dinucléaire comprend un groupe fluorophore, tel que, par exemple, la phycoerythrine (PE) ou la fluorescéine, en particulier l'isothiocyanate de fluorescéine (FITC).

Des composés préférés de formule (I) ou (II) sont ceux dans lesquels :
- M représente Zn ou Cu
- X représente -(CH₂)ₘ-NH-A où m= 1, A représente H, n=1 et p=0 ;
- X représente -(CH₂)ₘ-NH-A où m= 1, A représente H, n=2 et p=0 .
- X représente-CH₂-NHC(O)-R-NH-A, A représente H, n=1 et p=0.

Lorsque p=0, le groupe pyridine n'est pas substitué par Y.

L'invention concerne également, selon un autre aspect, les ligands utilisables comme intermédiaires dans la synthèse des complexes métalliques dinucléaires de formule (I) ou (II).

L'invention a donc également pour objet les composés de formule (III) ci-dessous 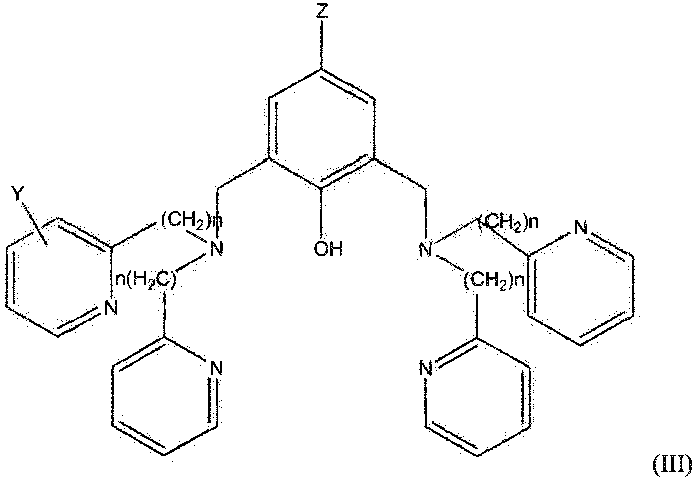 dans laquelle
- Z représente NH₂ ou un groupe -(CH₂)ₘ-NH-A, ou un groupe -CH₂-NHC(O)-R-NH-A dans lequel R est un groupe alkyle en C₂-C₁₀, de préférence en C₆-C₁₀, linéaire ou ramifié et A est H ou un groupe fluorophore, sous réserve que, lorsque m= 2, A soit différent de H
- m=1 à 12
- n = 1, 2 ou 3
- Y représente (CH₂)ₚ-NH₂ où p = 0 à 12.

Selon un aspect de l'invention, le bras greffé sur l'unité phénol du complexe métallique dinucléaire comprend un groupe fluorophore, tel que, par exemple, la phycoerythrine (PE) ou la fluorescéine, en particulier l'isothiocyanate de fluorescéine (FITC).

Des composés préférés de formule (III) sont ceux dans lesquels :
- Z représente -(CH₂)ₘ-NH-A où m= 1, A représente H, n=1 et p=0 ; ou
- Z représente -(CH₂)ₘ-NH-A où m= 1, A représente H, n=2 et p=0 ; ou
- Z représente-CH₂-NHC(O)-R-NH-A, A représente H, n=1 et p=0.

Ces composés peuvent être préparés selon les étapes a) et b) du procédé indiqué plus haut.

Selon un aspect ultérieur, l'invention concerne une méthode de détection et/ou de caractérisation de microparticules cellulaires, qui comprend la mise en contact d'un composé de formule (I) ou (II) 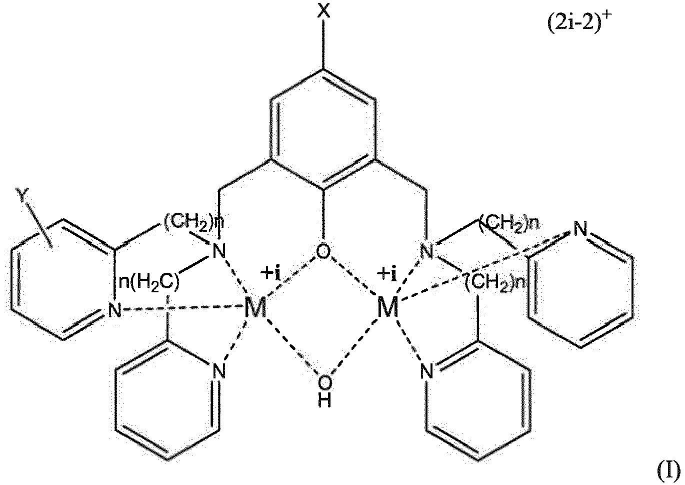 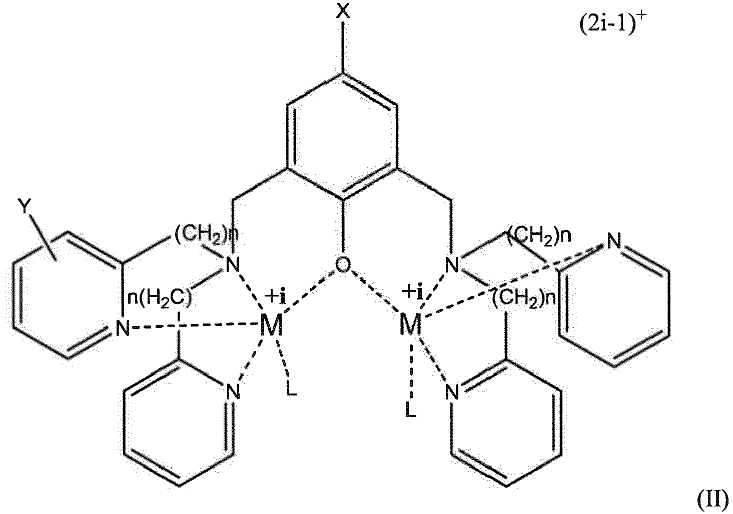 telles que définies ci-dessus, avec un échantillon de fluide biologique susceptible de contenir des microparticules cellulaires et la détection et/ou la caractérisation des microparticules capturées par ledit composé de formule (I) ou (II).

Si nécessaire, les phosphates organiques et inorganiques éventuellement présents dans le fluide biologique, notamment dans le plasma sanguin, peuvent être chélatés par des agents chélatants, tels que par exemple des sels de magnésium ou de lanthane.

Par « détection des microparticules cellulaires », on entend la détermination qualitative et /ou quantitative de la présence desdites microparticules cellulaires dans le fluide biologique.

Par « caractérisation des microparticules cellulaires », on entend l'identification, notamment le typage, en particulier le phénotypage desdites microparticules cellulaires, ainsi que, notamment, la détection de biomolécules éventuellement présentes sur les microparticules, la détection d'activités enzymatiques et la détermination des ARN.

Selon un aspect préféré, le composé de formule (I) ou (II) est, dans une première étape, immobilisé sur un support solide.

A titre d'exemples non-limitatifs, un tel support peut être choisi parmi le groupe constitué des plaques de microtitration, de feuilles, de cônes, de tubes, de puits, de billes, de particules ou encore de bandelettes.

L'invention concerne ainsi une méthode de détection et/ou de caractérisation de microparticules cellulaires, qui comprend les étapes suivantes :
- immobilisation d'un composé de formule (I) ou (II) telle que définie ci-dessus sur la surface du support solide,
- mise en contact avec un échantillon de fluide biologique susceptible de contenir des microparticules cellulaires
- capture des microparticules cellulaires sur ledit composé de formule (I) ou (II), et
- détection et/ou caractérisation des microparticules cellulaires capturées.

Selon une variante préférée, on utilise un support solide dont la surface a été préalablement activée, tel que, par exemple, une plaque de microtitration.

Selon cette variante, ladite méthode de détection et/ou de caractérisation de microparticules cellulaires comprend les étapes suivantes :
- activation d'un support solide,
- immobilisation d'un composé de formule (I) ou (II) telle que définies ci-dessus sur la surface du support activé,
- mise en contact avec un échantillon de fluide biologique susceptible de contenir des microparticules cellulaires
- capture des microparticules cellulaires sur ledit composé de formule (I) ou (II), et
- détection et/ou caractérisation des microparticules cellulaires capturées.

L'activation du support peut être réalisée par des méthodes usuelles, telles que, par exemple en utilisant le polyglutaraldéhyde. Sa structure polymérique permet une adsorption stable du polyglutaraldéhyde sur le support. Le glutaraldéhyde comporte des sites réactifs qui réagissent avec les groupements aminés (i.e. -NH₂) en formant des liaisons covalentes. L'étape d'activation comprend la formation de liaisons covalentes entre l'un au moins des sites réactifs du polyglutaraldéhyde immobilisé sur le support solide et les fonctions -NH₂ du ligand. On peut utiliser comme activateur, par exemple, du N-hydroxysuccinimide.

Au cours de l'étape d'immobilisation, le composé de formule (I) ou (II) se fixe sur le support activé en formant des liaisons covalentes.

Le groupement aminé (i.e. NH₂) qui se trouve à l'extrémité du bras X permet la formation de liaisons imines en réagissant avec les groupements aldéhydes de la surface du support activé.

La capture des microparticules cellulaires peut être réalisée par incubation d'un fluide biologique susceptible de contenir des microparticules cellulaires avec les composés de formule (I) ou (II), par exemple, par mise en suspension dans un tampon HEPES (acide 4-(2-hydroxyéthyl)-1-pipérazine éthanesulfonique) puis incubation avec les capteurs à température ambiante.

Les microparticules capturées peuvent ensuite être révélées en utilisant par exemple des techniques de détection mettant en oeuvre des anticorps marqués spécifiques (analyse photométrique ou fluorométrique), un marquage enzymatique (analyse chromogénique) ou des techniques d'amplification telles que la PCR quantitative ou q-PCR.

Par exemple, on peut utiliser :
a) des anticorps marqués spécifiques, à savoir
   - soit des anticorps dirigés contre des antigènes spécifiques de la cellule d'origine à partir de laquelle sont libérées les microparticules cellulaires, appelés CD (pour « Cluster of Differentiation »), par exemple les anticorps CD14 pour les monocytes ou CD105 (endogline) pour les cellules endothéliales ;
   - soit des anticorps dirigés contre des biomolécules actives, par exemple des anticorps dirigés contre le facteur tissulaire (FT, activateur de la coagulation) ou des anticorps dirigés contre les activateurs du plasminogène de type tissulaire (tPA) ou de type urokinase (uPA). Ces molécules ne sont pas exclusives d'un type cellulaire donné, mais il est important de savoir sur le plan physiopathologique si elles sont présentes sur la microparticule ;

   Les anticorps peuvent être marqués par une enzyme, telle que, par exemple, la peroxydase puis détectés par analyse photométrique en utilisant un substrat adapté. Ces anticorps peuvent également être marqués par un fluorochrome puis détectés par fluorescence ;
b) un test chromogénique : ce test permet de détecter l'activité d'un enzyme à l'aide d'un substrat synthétique.
   Par exemple, les activateurs du plasminogène tPA ou uPA transforment le plasminogène en plasmine (enzyme fibrinolytique et thrombolytique) et cette plasmine peut être détectée à l'aide d'un substrat chromogénique. Ainsi, en ajoutant aux microparticules capturées du plasminogène et un substrat synthétique chromogénique sensible à la plasmine, on pourra détecter l'activité du tPA et de l'uPA. Cette technique permet de détecter les molécules ayant une activité enzymatique se trouvant à la surface des microparticules.
c) la technique de q-PCR : les ARNm et microARN présents à l'intérieur des microparticules capturées peuvent être détectés via des amorces spécifiques. Les ARN sont libérés par éclatement des microparticules à l'aide d'un gradient de température lors du premier cycle de température de la q-PCR, puis amplifiés spécifiquement à l'aide des amorces avec lesquels ils sont mis en contact sur le support et de l'action de la polymérase.

Selon une variante de la méthode de détection mentionnée ci-dessus, on immobilise sur le support solide tel que défini plus haut un composé de formule (III) tel que défini plus haut, c'est-à-dire un ligand, et on incube l'ensemble support-ligand en présence d'ion métallique, de manière à ce que le complexe dinucléaire métallique de formule (I) ou (II) se forme *in situ.*

Dans cette variante, on peut, par exemple, immobiliser le ligand de formule (III) sur le support solide par incubation pendant une durée d'environ 2h à la température ambiante, de l'ordre de 20°C à 25°C, puis éliminer l'excès de ligand, et incuber l'ion métallique avec l'ensemble support-ligand pendant environ 2h à la même température.

La méthode de détection comprend ensuite les étapes mentionnées plus haut, à savoir
- la mise en contact dudit composé de formule (I) ou (II) avec un échantillon de fluide biologique susceptible de contenir des microparticules cellulaires,
- la capture des microparticules cellulaires sur ledit composé de formule (I) ou (II), et
- la détection et/ou la caractérisation des microparticules cellulaires capturées.

Selon un autre aspect de l'invention, le composé de formule (I) ou (II) est utilisé en solution.

On utilisera alors, de préférence, un composé de formule (I) ou (II) dans laquelle X représente -(CH₂)ₘ-NH-A, -CH₂-NHC(O)-R-NH-A où R est un groupe alkyle en C₂-C₁₀, de préférence en C₆-C₁₀, substitué ou non substitué, linéaire ou ramifié et A est un groupe fluorophore.

Les microparticules capturées peuvent, par exemple, être détectées par mesure de fluorescence à l'aide d'anticorps spécifiques, éventuellement marqués par des fluorophores

L'invention concerne également, selon un aspect ultérieur, une méthode de détection et/ou de caractérisation de microparticules cellulaires permettant l'évaluation du risque de développement et/ou le suivi thérapeutique de diverses pathologies, notamment thrombotiques, inflammatoires et/ou métaboliques, ou encore de maladies ou accidents cardiovasculaires ou neurovasculaires, ou de maladies comme le diabète, le cancer, la maladie d'Alzheimer ou d'autres pathologies.

Avantageusement, ladite méthode de détection et/ou de caractérisation de microparticules cellulaires permettant l'évaluation du risque de développement et/ou le suivi thérapeutique de diverses pathologies comprend les étapes suivantes :
- la mise en contact d'un composé de formule (I) ou (II) 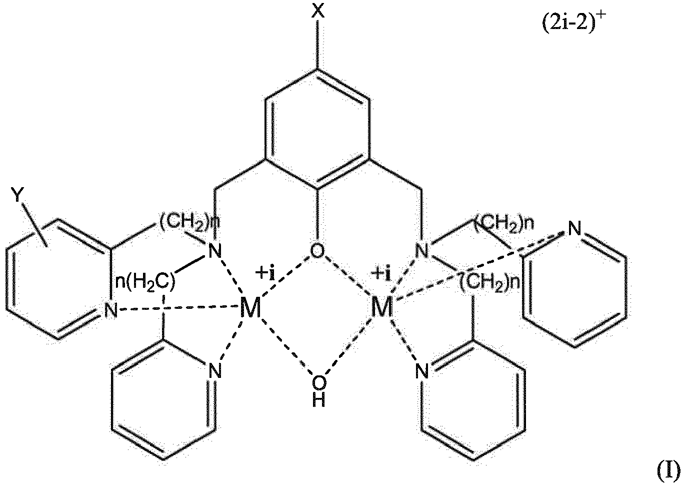 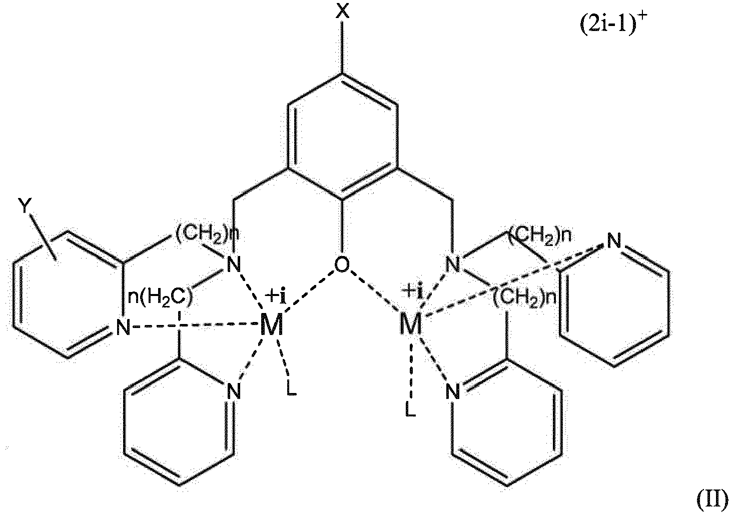 telles que définies ci-dessus avec un échantillon de fluide biologique susceptible de contenir des microparticules cellulaires,
- la détection et/ou la caractérisation des microparticules capturées par ledit composé de formule (I) ou (II), et
- la comparaison du résultat de la mesure effectuée à l'étape précédente avec le résultat d'une mesure identique réalisée sur un échantillon de fluide biologique témoin.

L'invention concerne également une méthode de diagnostic, d'évaluation du risque de développement et/ou de suivi thérapeutique de diverses pathologies, notamment thrombotiques, inflammatoires et/ou métaboliques, ou encore de maladies ou accidents cardiovasculaires ou neurovasculaires, ou de maladies comme le diabète, le cancer, la maladie d'Alzheimer ou d'autres pathologies comprenant les étapes suivantes :
- la mise en contact d'un composé de formule (I) ou (II) telles que définies ci-dessus avec un échantillon de fluide biologique susceptible de contenir des microparticules cellulaires,
- la détection et/ou la caractérisation des microparticules capturées par ledit composé de formule (I) ou (II), et
- la comparaison du résultat de la mesure effectuée à l'étape précédente avec le résultat d'une mesure identique réalisée sur un échantillon de fluide biologique témoin.

Les conditions de mise en contact du composée de formule (I) ou (II) avec les microparticules cellulaires, ainsi que de capture, détection et/ou caractérisation desdites microparticules cellulaires sont telles que définies ci-dessus.

Avantageusement, le fluide biologique témoin est un fluide biologique identique à celui testé, mais provenant d'un individu considéré comme sain. Alternativement, le fluide biologique témoin provient du même individu que le fluide biologique testé, mais résulte d'un prélèvement antérieur.

Des utilisations potentielles de la méthode de détection et/ou de caractérisation de microparticules cellulaires selon l'invention sont, par exemple, le diagnostic précoce et le suivi thérapeutique d'un risque thrombotique, pouvant causer un infarctus du myocarde ou un accident vasculaire cérébral, ou de maladies comme le diabète, le cancer, la maladie d'Alzheimer ou d'autres pathologies.

L'invention est illustrée de manière non limitative par les exemples suivants.

### Exemple 1 : préparation du ligand de formule (III)

### 4-(aminométhyl)-2,6-bis[[bis(2-pyridylméthyl)amino]méthyl]phénol HBPMP-NH₂

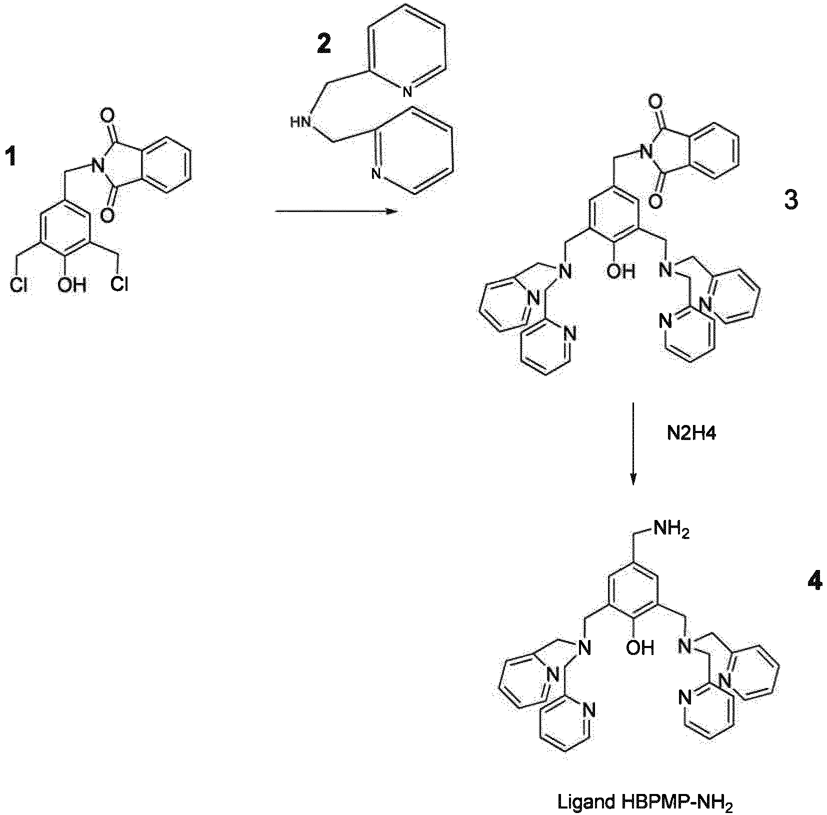

### 1) 2-{4-hydroxy-3,5-bis[[bis(2-pyridylméthyl)amino]méthyl]benzyl}isoindole-1,3-dione, (3)

Le composé **1** est décrit dans la publication M. Johansson et al., Inorg. Chem., 2003, 42, 7502-7511.

Dans un ballon tricol de 500 mL sous atmosphère inerte d'argon et plongé dans un bain de glace, on introduit 6,5 g (1 equiv.) du précurseur dichloré (**1**) dans 120 mL de THF sec. On place dans l'ampoule à addition 60 mL de THF sec dans lesquels ont été dissous 6,9 g (2 equiv.) de BPA (composé **2**) ainsi que 10 mL de triéthylamine (4 equiv.). L'ajout se fait à un goutte-à-goutte à 0 °C.

A la fin de l'ajout, la suspension marron obtenue est laissée sous agitation à température ambiante pendant 24h. Après filtration et rinçage au THF, on récupère le filtrat que l'on évapore.

Le solide obtenu est purifié sur colonne d'alumine neutre (hauteur : 11 cm ; diamètre : 4,5 cm) désactivée par un mélange de 1% d'H₂O, 10 mL de MeOH, 150 mL d'EtOAc et 200 mL de cyclohexane. Les solvants d'élution sont : EtOAc + cyclohexane 1/2, v/v, EtOAc + cyclohexane 1/1, v/v, EtOAc et EtOAc avec de 1 à 2% de MeOH.

Après évaporation, on récupère 7,85 g (66%) de produit 3 pur sous la forme d'un solide blanc.
RMN ¹H (300 MHz, CD₃CN, *δ* ppm) : 11,15 (s, 1H, OH) ; 8,43 (d, 4H, Py-N-C*H*) ; 7,83 (m, 4H, Pht) ; 7,59 (td, 4H, Py) ; 7,41 (d, 4H, Py-C-C*H*) ; 7,20 (s, 2H, Ar-*H*) ; 7,15 (m, 4H, Py-N-CH-C*H*) ; 4,46 (s, 2H, Pht-C*H*₂) ; 3,84 (s, 8H, Py-C*H*₂) ; 3,77 (s, 4H, Ar-C*H*₂-N).
RMN ¹³C (75 MHz, CD₃CN, δ ppm) : 168,3 (C=O) ; 159,4 (Py-*C*) ; 155,9 (*C*-OH) ; 149,0 (Py-N-*C*H) ; 136,8 (Py) ; 134,1 (Py) ; 132,5 (CO-*C*) ; 129,8 (Py-C-*C*H) ; 126,6 (Ar-*C*-CH₂) ; 124,6 (Ar-*C*-CH₂) ; 123,5 (Pht-*C*H) ; 123,3 (Pht-*C*H) ; 122,2 (Ar-*C*H) ; 60,1 (Ar-*C*H₂-Pht) ; 54,8 (Py-*C*H₂) ; 41,6 (Ar-*C*H₂-N) .
Spectre de masse ESI, *m*/*z* = 676 (M+H)⁺.

### 2)4-(aminométhyl)-2,6-bis[[bis(2-pyridylméthyl)amino]méthyl]phénol (4)

Dans un ballon de 500 mL surmonté d'un réfrigérant, on introduit 6.5 g du produit protégé (3) (1 equiv.) et 250 mL d'éthanol absolu. 20,5 mL d'hydrazine monohydratée (4 equiv.) dans 10 mL d'éthanol absolu sont ajouté au goutte-à-goutte, puis le milieu réactionnel est chauffé à reflux et laissé sous agitation pendant deux heures puis pendant une nuit à température ambiante.

La suspension blanche obtenue est filtrée, rincée à l'éthanol et le filtrat est évaporé. Le résidu est repris dans 150 mL d'une solution de soude à 2 N et laissée sous agitation deux heures. Le milieu réactionnel est neutralisé avec une solution de HCl 4N jusqu'à pH 7. La solution est extraite trois fois avec du dichlorométhane, puis séchée sur Na₂SO₄ et évaporée.

On obtient 5 g (95 %) du composé **4** sous la forme d'un solide blanc.
RMN ¹H (300 MHz, CDCl₃, *δ* ppm) : 8,55 (d, 4H, Py-N-C*H*) ; 7,57 (t, 4H, Py) ; 7,45 (d, 4H, Py-N-C-C*H*) ; 7,16 (s, 2H, Ar-*H*) ; 7,10 (t, 4H, Py) ; 5,29 (s, 1H, O*H)* ; 3,98 (s, 2H, NH₂-C*H*₂) ; 3,84 (d, 8H, Py-C*H*₂) ; 3,77 (s, 4H, Ar-C*H*₂-N) ; 2,89 (s, 2H, N*H*₂).
RMN ¹³C (75 MHz, CDCl₃, *δ* ppm) : 159,6 (Py-*C*) ; 155,3 (*C*-OH) ; 148,8 (Py-N-*C*H) ; 136,6 (Py-N-C-CH-*C*H) ; 130,0 (Ar-*C*-CH₂-NH₂) ; 129,0 (Ar-*C*H) ; 124,1 (Ar-*C*-CH₂-N) ; 123,1 (Pyr-C-*C*H) ; 122,0 (Py-N-CH-*C*H) ; 59,8 (Py-*C*H₂) ; 54,7 (Ar-*C*H₂-N) ; 45,1 (NH₂-*C*H₂).
Spectre de masse ESI, *m*/*z* = 568 (M+Na)⁺, 546 (M+H)⁺.

### Exemple 2 : préparation du complexe [µ-[4-(aminométhyl)-2,6-bis[[bis(2-pyridylméthyl)amino]méthyl]-phénolato]]-µ-hydroxo, dizinc, diperchlorate. (5)[(Zn₂)(BPMP-NH₂)(µ-OH)]·(ClO₄)₂.

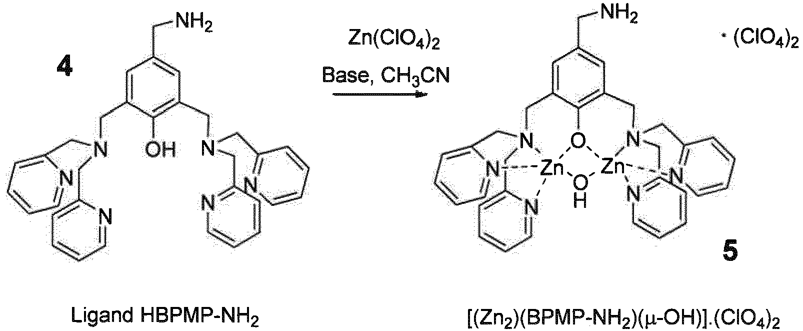

Dans un ballon de 25 mL on introduit le ligand **4** (0,4 g, 1 equiv.) dissous dans 8 mL d'acétonitrile et 0.155 g (2,1 equiv.) de triéthylamine.

On y ajoute 0,57 g (2,1 equiv.) de perchlorate de zinc hexahydraté dissous dans 6 mL d'acétonitrile au goutte-à-goutte. A la fin de l'ajout du perchlorate, on laisse sous agitation à température ambiante. On évapore partiellement la solution de manière à avoir un volume d'environ 5 mL puis on rajoute de l'éther éthylique jusqu'à observer un trouble et on laisse à -20°C pendant une nuit.

On obtient un précipité qui est filtré, rincé à l'éther éthylique et séché. On resoumet le filtrat au même traitement, ce qui permet au final de récupérer 280 mg (43%) du composé **5** sous la forme d'une poudre jaune pâle.
RMN ¹H (300 MHz, CD₃CN, *δ* ppm) : 8,98 (d, 4H, Py-*H*) ; 8,05 (t, 4H, Py-*H*) ; 7,65 (t, 4H, Py-*H*) ; 7,53 (d, 4H, Py-*H*) ; 7,09 (s, 2H, Ar-*H*) ; 4,01 (s, 8H, Py-C*H*₂) ; 3,91 (d, 2H, NH₂-C*H*₂) ; 3,84 (s, 4H, Ar-C*H*₂-N).
RMN ¹³C (75 MHz, CD₃CN, *δ* ppm) : 163,4 (Ar-CO) ; 156,5 (Py) ; 149,5 (Py) ; 142,5 (Py) ; 133,8 (Ar-*C*-CH₂-NH₂) ; 126,0 (Py) ; 125,9 (Ar) ; 125,3 (Ar) ; 123,2 (Py-N-CH-*C*H) ; 61,0 (NH₂-*C*H₂) ; 59,0 (Ar-*C*H₂-N) ; 58,1 (Py-*C*H₂).
Spectre de masse ESI, *m*/*z* = 791 (M-ClO₄)⁺.

### Exemple 3 : préparation du ligand de formule (III) 6-amino-N-(3,5-bis((bis(2-pyridylméthyl)amino)méthyl)-4-hydroxybenzyl)hexanamide (8) HBPMP-C₆NH₂

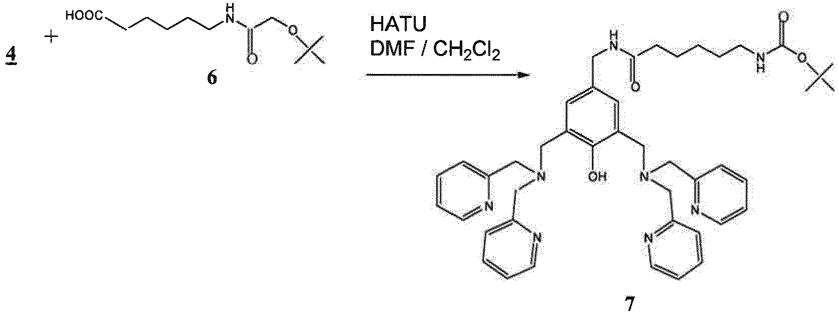

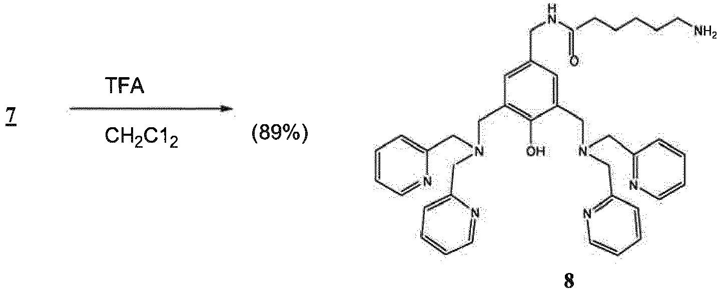

Le composé **6** est décrit dans la publication M. B. Doughty et al., 1993, J. Med. Chem, 36, 272-279.

### 1) 6-tert-butyl-N-(3,5-bis((bis(2-pyridylméthyl)amino)méthyl)-4-hydroxybenzylamino)-6-oxohexylcarbamate (7)

Dans un ballon tricol de 250 mL séché à l'étuve, surmonté d'une ampoule à addition et d'un ballon d'argon et placé dans un bain de glace on introduit 835 mg (1,2 equiv.) d'acide 6-tert-butoxycarbonylamino-hexanoïque et 40 mL de dichlorométhane sec. On ajoute 1,37 g (1,2 equiv.) de HATU (2-(7-Aza-1H-benzotriazole-1-yl)-1,1,3,3-tetraméthyluronium hexafluorophosphate) dans 6 mL de DMF(diméthylformamide) et 1,5 mL (3 equiv.) de triéthylamine (NEt₃) avant de laisser 30 minutes sous agitation (toujours dans le bain de glace). On rajoute le ligand **4** (1,52 g, 1 equiv.) dissous dans 40 mL de dichlorométhane sec) au goutte-à-goutte. On laisse agiter à 0°C pendant une heure puis à température ambiante pendant une nuit et une journée.

Après évaporation du dichlorométhane et du DMF sous vide, on obtient 4,6g d'une huile épaisse qui est purifié par chromatographie sur colonne d'alumine neutre (hauteur : 5,5 cm ; diamètre : 4,5 cm). La colonne est désactivée par une solution de 2 gouttes d'eau distillée avec 10 mL de MeOH, 300 mL d'EtOAc et 150 mL de cyclohexane. Les solvants d'élution sont : EtOAc + cyclohexane, EtOAc, EtOAc + de 1 à 5% de MeOH.

Après évaporation on récupère 1, 9 g d'un produit blanc collant. Il est repris dans de l'eau, gratté, décanté et extrait par du dichlorométhane. Après séchage sur Na₂SO₄, filtration et évaporation on récupère 1,7 g (80%) du composé **7** sous la forme d'un solide beige.
RMN ¹H (300 MHz, CDCl₃, TMS, *δ* ppm) : 11,12 (s, 1H, O*H*) ; 8,53 (m, 4H, Py) ; 7,60 (m, 7H, Py) ; 7,48 (m, 5H, Py) ; 7,27 (m, 2H Ar) ; 5,79 (s, 1H, NH) ; 4,32 (d, 2H, Ar-C*H*₂₋NH) ; 4,13 (d, 4H, Ar-C*H*₂-N) ; 3,86 (s, 10H, Py-C*H*₂) ; 2,16 (t, 2H, C*H*₂) ; 1,86 (d, 5H, C*H*₂) ; 1,64 (t, 3H, C*H*₂) ; 1,42 (s, 9H, tBu).
RMN ¹³C (75 MHz, CDCl₃, TMS, *δ* ppm) : 172,8 (CH₂-*C*=O-NH) ; 159,4 (O-*C*=O) ; 155,8 (Ar-*C*-OH) ; 149,2 (Py-N-*C*H) ; 136,9 (Py) ; 129,3 (Ar-CH) ; 128,3 (O-*C*-(CH₃)₃) ; 124,6 (Ar-*C*-CH₂-NH) ; 123,3 (Py-C-*C*H) ; 122,3 (Py-N-CH-*C*H) ; 60,1 (Py-*C*H₂) ; 55,0 (Ar-*C*H₂) ; 43,7 (O-CO-NH-*C*H₂) ; 36,9 (NH-CO-*C*H₂-CH₂) ; 30,1 (*C*H₂) ; 28,8 (*C*H₃) ; 26,7 (*C*H₂) ; 25,6 (*C*H₂).
Spectre de masse ESI, *m*/*z* = 781 (M+Na)⁺, 759 (M+H)⁺

### 2) 6-amino-N-(3,5-bis((bis(2-pyridylméthyl)amino)méthyl)-4-hydroxybenzyl-hexanamide (8)

Dans un ballon de 250 mL surmonté d'un réfrigérant, on introduit 1,69 g du produit protégé (7) (1 equiv.) et 200 mL de dichlorométhane sec. Le mélange est refroidi par un bain de glace puis 9 mL d'acide trifluoroacétique (46 equiv.) dans 10 mL de dichorométhane sec sont additionné au goutte-à-goutte. Le milieu réactionnel est laissé sous agitation pendant une nuit à température ambiante.

La solution obtenue est neutralisée jusqu'a pH 9 par une solution de soude 2N puis par une solution de NaHCO₃ saturée. Le mélange est extrait trois fois avec du dichlorométhane, puis séché sur Na₂SO₄, filtré et évaporé. On obtient une poudre qui est solubilisée à chaud dans l'acétonitrile. Après filtration à chaud, la solution est évaporée et on obtient 1,2 g (82%) du composé **8** sous la forme d'un solide jaune pâle.
RMN ¹H (300 MHz, CDCl₃, TMS, *δ* ppm) ; 8,51 (d, 4H, Py) ; 7,56 (t, 4H, Py) ; 7,37 (d, 4H, Py) ; 7,13 (t, 4H, Py), 7,04 (s, 2H Ar) ; 4,24 (d, 2H, Ar-*CH₂*-NH) ; 3,83 (s, 8H, Py-C*H*₂) 3,70 (s, 4H, Ar-C*H*₂-N) ; 2,90 (t, 2H, C*H*₂) ; 2,24 (t, 2H, C*H*₂) ; 1,75 (t, 2H, C*H*₂) ; 1,66 (t, 2H, C*H*₂) ; 1.26(t, 2H, C*H*₂)
RMN ¹³C (75 MHz, CDCl₃, TMS, *δ* ppm) : 172,9 (-*C*=O) ; 158,6 (Py-N-*C*H), 155,0 (Ar-*C*-OH) ; 148,8 (Py); 136,7 (Py) ; 129,4 (Ar-*C*H) ; 128,8 (Ar-*C*H) ; 122,2 (Py); 123,3 (Py) ; 122,3 (Py) ; 59,9 (Py-*C*H₂) ; 54,7 (Ar-*C*H₂) ; 42,9 (Ar-*C*H₂-NH-) ; 39,7 (-*C*H₂-NH₂) ; 35,6 (*C*H₂) ; 27,6 (*C*H₂) ; 25,3 (*C*H₂) ; 24,8 (*C*H₂).
Spectre de masse ESI, *m*/*z* = 681 (M+Na)⁺, 659 (M+H)⁺, 330 (M+2H)²⁺.

### Exemple 4 : préparation du complexe [µ-[6-amino-N-(3,5-bis((bis(2-pyridylméthyl)amino)-méthyl)-4-hydroxybenzyl)hexanamide]]-µ-hydroxo, dizinc, diperchlorate. (9) [(Zn₂)(BPMP-C₆NH₂)(µ-OH)]·(ClO₄)₂.

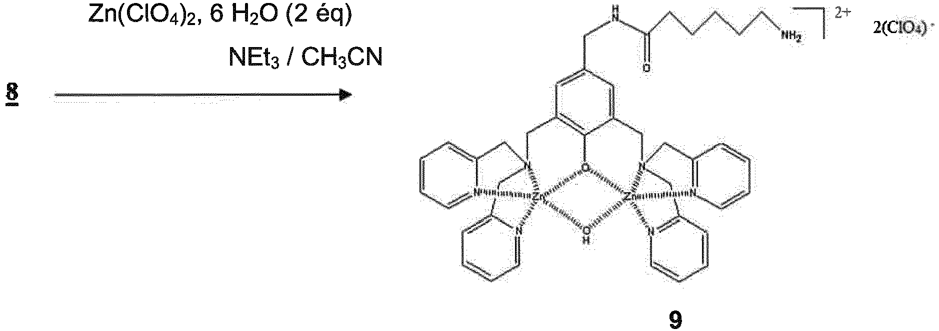

Dans un ballon de 100 mL on introduit le ligand **8** (0,4 g, 1 equiv.) dissous dans 10 mL d'acétonitrile (au chauffe un peu pour tout dissoudre) et 0,13g (2,1 equiv.) de triéthylamine.

On y ajoute 0,9g (2,1 equiv.) de perchlorate de zinc hexahydraté dissous dans 4 mL d'acétonitrile au goutte-à-goutte. A la fin de l'ajout du perchlorate, on laisse sous agitation à température ambiante pendant 2 heures. On évapore partiellement la solution de manière à avoir un volume d'environ 5 mL puis on rajoute de l'éther éthylique jusqu'à observer un trouble et on laisse à -20 °C pendant une nuit.

On obtient un précipité qui est filtré, rincé à l'éther éthylique et séché. On resoumet le filtrat au même traitement, ce qui permet au final de récupérer 270 mg (43%) du composé **9** sous la forme d'un solide jaune pâle.
RMN ¹H (300 MHz, DMSO, *δ* ppm) : 8,96 (d, 4H, Py-*H*) ; 8,80 (s, 1H, OH), *Py-H)* ; 8,11 (t, 4H, Py-*H*) ; 7,68 (t, 4H, Py-*H*), 7,59 (d, 4H, Py-*H*) ; 6,83 (s, 2H, Ar-*H*) ; 6,64 (s, 1H, N*H*-CO) ; 4,01 (s, 8H, Py-C*H*₂) ; 3,91 (d, 2H, NH₂-C*H*₂) ; 3,84 (s, 4H, Ar-C*H*₂-N) ; 4,11 (m, 12H, C*H*₂-N); 2,12 (m, 2H, -C*H*₂), 1,52 (m, 4H, -C*H*₂) ; 1,29 (m, 4H, -C*H*₂).
RMN ¹³C (75 MHz, DMSO, *δ* ppm) : 171,4 (-*C*=O) ; 155,0 (Py-N-*C*) ; 154,9 (Ar-*C*-OH), 147,5 (Py-*C*H) ; 140,8 (Py) ; 140,2 (Ar-*C*-CH₂-NH₂) ; 130,8 (Ar-*C*H) ; 129,8 (Ar-*C*H) ; 126,0 (Ar-C) ; 126,3 (Ar-C) ; 124,6 (Py-*C*H) ;123,7 (*C*H) ; 122,9 (*C*) ; 121,1 (*C*H) ; 58,8 (Ar-*C*H₂-NH-CO) ; 57,7 (Ar-*C*H₂-N); 56,7 (Py-*C*H₂), 41,1 (*C*H₂) ; 35,4 (*C*H₂) ; 29,7 (*C*H₂) ; 26,0 (*C*H₂) ; 25,4 (*C*H₂) ; 24,6 (*C*H₂).
Spectre de masse ESI, *m*/*z* = 905 (M-ClO₄)⁺, 403 (M-2ClO₄)²⁺.

### Exemple 5 : dosage colorimétrique de microparticules cellulaires par immobilisation du complexe de l'exemple 4 sur une plaque de microtitration (étude de la sensibilité)

### a) Réactifs :

- Glutaraldéhyde 25%
- Ethanolamine à 16.5 M
- Tampon bicarbonate (Bic Na), pH 9,5 à 1M, à faire uniquement par titration d'une solution de NaH₂CO₃ (pH 8,3) avec une solution de Na₂CO₃ pH 11,6.
- Tampon HEPES 10 mM pH 7,4; 0,15 M NaCl)
- (hBPMP/Zinc)-C₆NH₂ à 150µM

### b) Protocoles :

- Polymérisation du glutaraldéhyde : On prépare 50 ml d'une solution de glutaraldéhyde à 2,5% dans le Bic Na 0,1M, puis on place le mélange dans un flacon fermé à 37°C pendant 2h.
- Adsorption du polyglutaraldéhyde sur la surface de chlorure de polyvinyle :
   On utilise une plaque de microtitration à 96 puits.
   On dépose 100µL par puits de glutaraldéhyde 2,5% et on laisse 2h à 22°C. On élimine le polyglutaraldéhyde et on rince la surface 3 fois à l'eau.
- Immobilisation du complexe [(Zn₂)(BPMP-C₆NH₂)(µ-OH)]·(ClO₄)₂ Le complexe dans HEPES 0,1 M, pH 8,2 est immobilisé sur la surface de la plaque en chlorure de polyvinyle pendant 24h à +22°C:
   Les groupements aldéhydes libres sont saturés à l'éthanolamine 0,3M par incubation de la surface avec cette solution pendant 2h à 22°C, puis on élimine l'éthanolamine par lavage à l'eau puis 3 lavages en tampon HEPES 10 mM pH 7,4, 0,15 M NaCl. Les plaques sont conservées à 4°C .
- Une solution tampon (HEPES 10mM, NaCl 0,9%) contenant les microparticules est incubée pendant 12h, puis les plaques sont rincées avec la solution tampon. Les microparticules sont issues de l'activation de culture cellulaire de cellules endothéliales microvasculaires humaines HMEC1 par du TNF-α, comme décrit dans R. Lacroix et al., Blood, 2007, 110, 2432-2439.

### c) Détection

La révélation est effectuée par analyse photométrique. On incube avec un anticorps anti-CD105 spécifique des microparticules étudiées pendant 3 h, puis on effectue un rinçage à l'HEPES 10mM + BSA 40mg/mL.

On incube ensuite avec l'anticorps secondaire couplé à la peroxydase, puis on effectue un rinçage à l'HEPES 10mM + BSA 40mg/mL.

La révélation est effectuée avec de l'ABTS (acide 2,2'-azino-bis(3-éthylbenzothiazoline-6-sulfonique) 1mg/ml si on utilise un anticorps secondaire couplé à la peroxydase.

La lecture est effectuée sous spectrophotomètre.

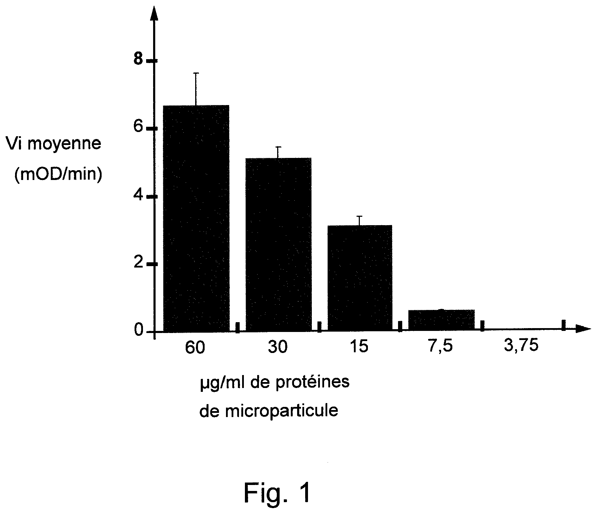

Les résultats sont rapportés sur la figure 1 qui représente la détection par analyse photométrique d'une dilution de microparticules rapportée à la quantité de protéines.

Le signal obtenu est exprimé en mOD/min correspondant à la vitesse initiale (Vi) de la réaction de dégradation du substrat par la peroxydase.

Le signal obtenu en fonction de la quantité de microparticules montre une dose-réponse spécifique.

### Exemple 6 : Etude de la spécificité des complexes de Zn des exemples 2 et 4 par méthode immunochimique sur une plaque de microtitration

Une solution de glutaraldéhyde à 2,5% diluée dans du Bic Na (0,1 M ; pH 9,5) est déposée dans chaque puits de la plaque de microtitration et laissée pendant 4h à 22°C. Après élimination du glutaraldéhyde et plusieurs rinçages à l'eau, le complexe à tester, à savoir [(Zn₂)(BPMP-NH₂)(µ-OH)]·(ClO₄)₂ (exemple 2) ou [(Zn₂)(BPMP-C₆NH₂)(µ-OH]·ClO₄)₂ (exemple 4), est incubé à la concentration de 500 nm (concentration déterminée pat titration). Puis, le complexe est éliminé par rinçage avec une solution HEPES 10mM. Enfin, les groupes aldéhydes libres sont saturés par une solution d'éthanolamine à 0,3M suivie de plusieurs rinçages au tampon HEPES 10mM .

Une solution tampon (HEPES 10mM, NaCl 0,9%) contenant des microparticules (issues de l'activation de culture cellulaire de cellules HMEC1) est ensuite incubée pendant 1h puis éliminée par rinçage avec la solution tampon.

On utilise une méthode immunochimique indirecte (anticorps primaire anti-CD105 à 1 µg/ml ; anticorps secondaire couplé à la peroxydase à 160 ng/ml et de l'ABTS à 1 mg/ml) pour analyser la présence de l'antigène endothélial CD105 sur les microparticules.

La lecture est effectuée sous spectrophotomètre à 405 nm.

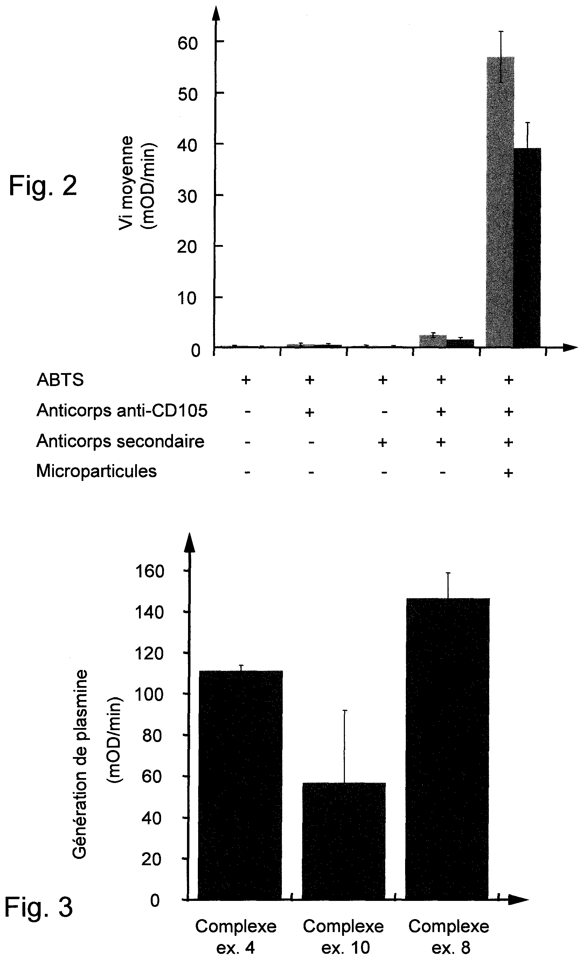

Les résultats sont rapportés sur la figure 2 qui représente la détection par analyse photométrique d'une dilution de microparticules rapportée à la quantité de protéines.

Le signal obtenu est exprimé en mOD/min correspondant à la vitesse initiale (Vi) de la réaction de dégradation du substrat par la peroxydase, et est représenté en gris foncé pour le complexe de l'exemple 2 et en gris clair pour le complexe de l'exemple 4.

La présence (signe +) ou l'absence (signe -) des réactifs dans le milieu de mesure est indiquée en dessous de l'axe des abscisses.

Le signal obtenu montre que le complexe selon l'invention reconnait spécifiquement les microparticules lors d'une révélation immunochimique ;

De plus, la méthodologie semble n'engendrer qu'un très faible bruit de fond, comme le montre le très faible signal mesuré en l'absence des microparticules.

### Exemple 7 : préparation du complexe [µ-[4-aminométhyl)-2,6-bis[[bis(2-pyridylméthyl)omino]méthyl]-phénolato]]-µ-hydroxo, dicuivrique, diperchlorate. (10) [(Cu₂)(BPMP-NH₂)(µ-OH)]·(ClO₄)₂.

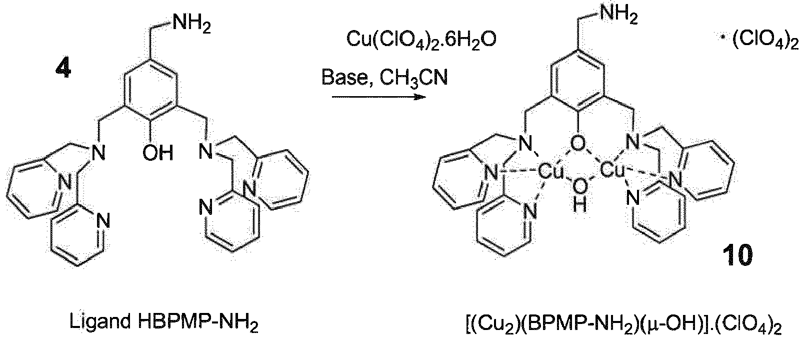

Dans un ballon de 25 mL on introduit le ligand **4** (0,146 g, 1 equiv.) dissous dans 10 mL d'acétonitrile et 77µl (2 equiv.) de triéthylamine.

On y ajoute 0,21 g (2 equiv.) de perchlorate cuivrique hexahydraté dissous dans 3 mL d'acétonitrile au goutte-à-goutte. A la fin de l'ajout du sel de cuivre, on laisse sous agitation à température ambiante 30 mn. On évapore partiellement la solution de manière à avoir un volume d'environ 5 mL puis on rajoute du tétrahydrofurane jusqu'à observer un trouble et on laisse à -20°C pendant trois jours.

On obtient un précipité qui est filtré puis rincé à l'éther éthylique. Le solide récupéré est remis en solution dans l'acétonitrile et reprécipité comme précédemment avec du tétrahydrofurane à -20°C. Après filtration, rinçage et séchage, on récupère 118 mg (36%) du composé **10** sous la forme d'une poudre verte.
Spectre de masse ESI, *m*/*z* = 806 (z = 1) [M-ClO₄⁻ + H₂O] ; 353.5 (z = 2) [M-2ClO₄⁻ + 2H₂O] ; UV/vis (DMSO/eau, pH =7; 30/70) : λₘₐₓ (ε) ≈ 340 (sh), 425 (sh), 700 (191 M⁻¹cm⁻¹).

### Exemple 8 : préparation du complexe [µ-[6-amino-N-(3,5-bis((bis(2-pyridylméthyl)amino)méthyl)-4-hydroxybenzyl)hexanamide]]-µ-hydroxo, dicuivrique, diperchlorate. (11) [(Cu₂)(BPMP-C₆NH₂)(µ-OH)]·(ClO₄)₂.

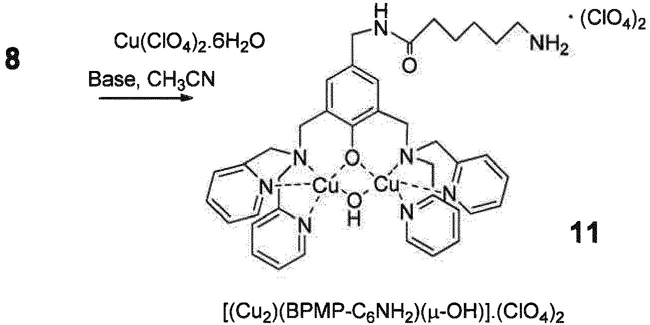

Dans un ballon de 25 mL, on introduit le ligand **8** (0,350 g, 1 equiv.) dissous dans 12 mL d'acétonitrile et 0,118 g (2,2 equiv.) de triéthylamine.

On y ajoute 0,43 g (2,1 equiv.) de perchlorate cuivrique hexahydraté dissous dans 8 mL d'acétonitrile au goutte-à-goutte. A la fin de l'ajout du sel de cuivre, on laisse sous agitation à température ambiante pendant 1 heure. On évapore partiellement la solution de manière à avoir un volume d'environ 5 mL puis on rajoute du tétrahydrofurane jusqu'à observer un trouble et on laisse à -20 °C.

Une huile épaisse se dépose. Le surnageant est éliminé et 5 ml d'éther éthylique sont ajouté et l'huile est grattée avec une baguette de verre. Une poudre verte précipite : elle est filtrée, rincée à l'éther éthylique et séchée. Le filtrat est remis à -20°C et une seconde fraction d'huile se dépose. Elle est traitée comme précédemment. Au final 246 mg (46%) du composé **11** sont récupérés sous la forme d'une poudre verte.
Spectre de masse ESI, m/z = 400 (z =2) [M-2ClO₄] ; UV/vis (DMSO/eau, pH = 7; 30/70): λₘₐₓ (ε) ≈ 350 (sh), 450 (316), 700 (202 M⁻¹cm⁻¹).

### Exemple 9 : préparation du ligand de formule (III)

### 6-amino-N-(3,5-bis((bis(2-pyridyléthyl)amino)méthyl)-4-hydroxybenzyl)-hexanamide (12) HBPEP-C₆NH₂

Le ligand 12 est préparé de manière similaire au ligand 8 en quatre étapes à partir du composé 1 sur lequel est condensé la bis(2-pyridyléthyl) amine dont la préparation est décrite dans la publication de M. A. Halcrow et al., 2003, Dalton Trans., 4224-4225.

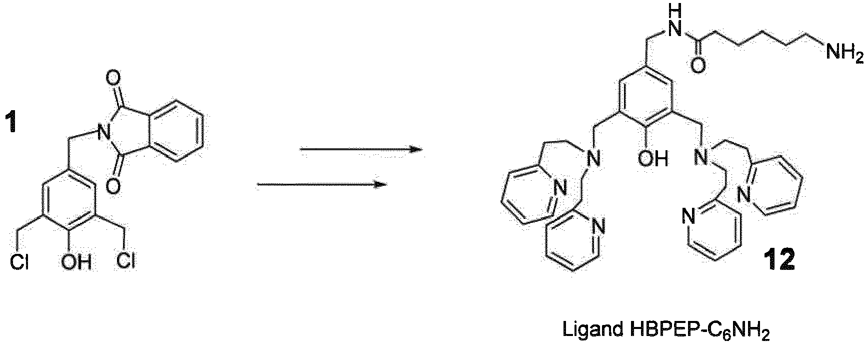

RMN ¹H (300, CDCl₃, *δ* ppm) : 8,42 (d, 4H, Py-*H*) ; 7,52 (td, 4H, Py-*H)* ; 7,07 (m, 8H, Py-*H*) ; 6.93 (s, 2H, Ar-*H*); 6,85 (t, 1H, N*H*-CO) ; 4,22 (d, 2H, Ar-C*H*₂-NH-) ; 3,80 (s, 4H, Ar-C*H*₂-N) ; 2,99 (m, 16H, Py-C*H*₂-C*H*₂-N) ; 2.79 (m, 2H, NH₂-C*H*₂); 2,16 (t, 2H, CO-C*H*₂), 1,58 (m, 4H, -C*H*₂) ; 1,32 (m, 2H, -C*H*₂).
RMN ¹³C (75 MHz, CDCl₃, *δ* ppm) : 172,9 (-*C*=O) ; 159,6 (Py-N-*C*) ; 155,3 (Ar-*C*-OH), 149,0 (Py-N-*C*H) ; 136,4 (Py-*C*) ; 128,6 (Ar-*C*-CH₂-NH) ; 128,4 Ar-*C*H ; 123,4 (Py-N-CH-*C*H) ; 122,8 (Ar-*C*-CH₂-N) ; 121,3 (Py-CH₂-*C*H₂) ; 54,7 (Ar-*C*H₂-NH); 53,3 (Py-CH₂-*C*H₂) ; 42,9 (Ar-*C*H₂-NH) ; 40,0 (NH₂-*C*H₂) ; 35,7 (CO-*C*H₂) ; 34,8 (Py-*C*H₂-*C*H₂); 28,3 (NH₂-CH₂-*C*H₂); 25,5 (*C*H₂); 24.8 (*C*H₂).
Spectre de masse ESI, *m*/*z* = 715 [M + H⁺]

### Exemple 10 : préparation du complexe [µ-[6-amino-N-(3.5-bis((bis(2-pyridyléthyl)amino)méthyl)-4-hydroxybenzyl)hexanamide]]-µ-hydroxo, dizinc, diperchlorate. (13) [(Zn₂)(BPEP-C₆NH₂)(µ-OH)]·(ClO₄)₂.

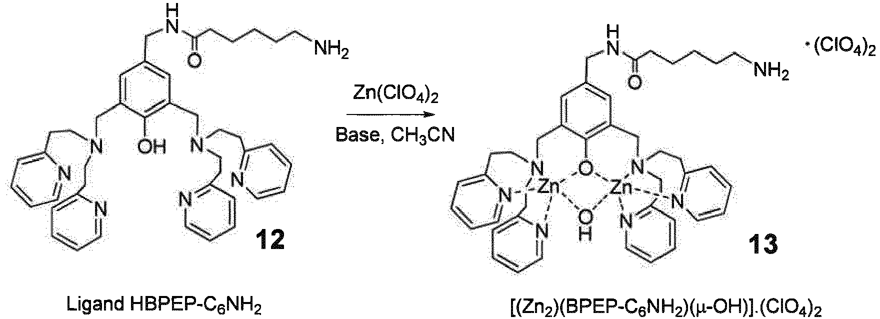

Dans un ballon de 50 mL on introduit le ligand **12** (551 mg, 0.77 mmol, 1 équiv.) dissous dans 15 mL d'acétonitrile et 280 µL (2,5 équiv.) de triéthylamine. On y ajoute au goutte-à-goutte 724 mg de perchlorate de zinc hexahydraté (2,5 équiv.) dissous dans 8 mL d'acétonitrile. A la fin de l'ajout dusel de zinc, on laisse sous agitation à température ambiante pendant 5 heures. On évapore partiellement la solution de manière à avoir un volume d'environ 5 mL puis on rajoute du tétrahydrofurane jusqu'à observer un trouble et on laisse à -20 °C pendant une nuit.

On obtient un précipité pâteux qui est filtré, rincé à l'éther éthylique et séché. On récupère un solide qui est gratté avec une baguette de verre en présence d'éther éthylique puis la solution est éliminée et on recommence trois fois l'opération. Au final, après séchage, on récupère 218 mg (27 %) du composé **13** sous la forme d'un solide jaune pâle.
RMN ¹H (400 MHz, CD₃CN + DMSO, *δ* ppm) : 8,48 (d, 4H, Py-*H*) ; 7,99 (m, 4H, Py*-H)* ; 7,45 (m, 8H, Py-*H*) ; 7.40 (s, 1H, N*H*-CO) ; 6,86 (s, 2H, Ar-*H*); 4,05 (s, 2H, Ar-C*H*₂-NH-) ; 3,62 (t, 4H, Ar-C*H*₂-N) ; 3,05 (m, 8H, Py-CH₂-C*H*₂-N) ; 2.79 (m, 10H, NH₂-C*H*₂, Py-C*H*₂); 2,09 (t, 2H, CO-C*H*₂), 1,56 (m, 4H, -C*H*₂) ; 1,30 (m, 2H, -C*H*₂).
RMN ¹³C (100 MHz, CD₃CN + DMSO, *δ* ppm) : 173,1 (-*C*=O) ; 161,6 (Py-N-*C*) ; 158,3 (Ar-*C*-OH), 149,7 (Py-N-*C*H) ; 141,8 (Py-*C*) ; 132,0 (Ar-*C-*CH₂-NH₂) ; 132,0 Ar-*C*H) ; 126,3 (Py-N-CH-*C*H) ; 124,7 (Py-N-C-*C*H) ;123,1 (Ar-*C*-CH₂-N) ; 47,3 (Py-CH₂-*C*H₂); 36,2 (Py-*C*H₂) ; 32,7 (CO-*C*H₂) ; 27,5 (NH₂-*C*H₂) ; 26,5 (CO-CH₂-*C*H₂) ; 25,6 (*C*H₂).
Spectre de masse ESI, *m*/*z;* 961 (z = 1) [M-ClO₄] ; 431 (z = 2) [M-2ClO₄]

### Exemple 11 : Etude de la sensibilité du système de révélation des microparticules par les complexes de Zn des exemples 4 et 10 et le complexe de Cu de l'exemple 8 par mesure de la génération de plasmine

Le principe de la mesure consiste à mesurer l'activité fibrinolytique résultant de la génération de plasmine par les microparticules cellulaires issues de l'activation de culture cellulaire de cellules HMEC1. Ces microparticules portent à leur surface de l'urokinase (ou enzyme activateur de plasminogène - uPA), qui permet la transformation du plasminogène en plasmine, laquelle lyse la fibrine.

La génération de plasmine est détectée par le clivage d'un substrat chromogénique, le méthylmalonyl-hydroxypropyl-arginyl-paranitroanilide (CBS) par la plasmine (mesure spectrophotométrique).

### a) Protocoles

### - Préparation de la plaque de microtitration

On utilise une plaque 96 puits en PVC à fonds en U.

On prépare un tampon de test de composition suivante :

| | Solution stock | Volume de stock pour 1 l de tampon | Solution finale |
|---|---|---|---|
| Phosphate pH = 7,4 | 0,5 M | 100 ml | 50 mM |
| NaCl | 5 M | 16 ml | 80 mM |
| BSA | 40g/l | 2g ou 50 ml à 40% | 2g/l |
| Tween 20 | 10% | 1 ml | 0,01% |
| NaN₃ ou thimérosal | 10% | 1ml | 0,01% |
| qsp H₂O | | Qsp H₂O 832 ml | |

On incube 150µL de tampon de test par puits pendant 30 min à température ambiante.

### - Préparation du plasminogène à 4 µM pour une plaque entière

On utilise 48,3 µl d'une solution mère de plasminogène à 103,5 µM et on ajoute 1201,7 µl de tampon de test.

### - Préparation du CBS0065 à 3 mM pour une plaque entière

On utilise une solution mère de CBS0065 à 7,5 mM et on ajoute 750 µl de tampon de test.

### b) Détection

La plaque contenant le tampon de test est renversée pour vider les puits, puis séchée avec du papier absorbant.

On mélange la solution de plasminogène à 4 µm avec la solution de CBS0065 à 3 mM, puis on incube 25µl d'une solution tampon (HEPES 10mM, NaCl 0,9%) contenant les microparticules (issues de l'activation de culture cellulaire de cellules HMEC1) pendant 1 h à 37°C. On ajoute 12,5 µl de CBS0065 et 12,5 µl de plasminogène (respectivement 2 µM et 1,5 mM final), et on scelle la plaque.

La lecture est effectuée sous spectrophotomètre à 405 nm et 490 nm à 37°C pendant 18h.

Les résultats sont rapportés sur la figure 3 qui représente la détection de la génération de plasmine par dosage de l'activité fibrinolytique

Le signal obtenu est exprimé en mOD/min.

Les résultats montrent que l'immobilisation des microparticules par les complexes métalliques selon l'invention permet leur dosage fonctionnel. On observe également une variation des résultats en fonction de la longueur du « bras » lié au groupe pyridine et du type d'ion métallique.

## Revendications

1. Composé de formule (I) ou de formule (II): 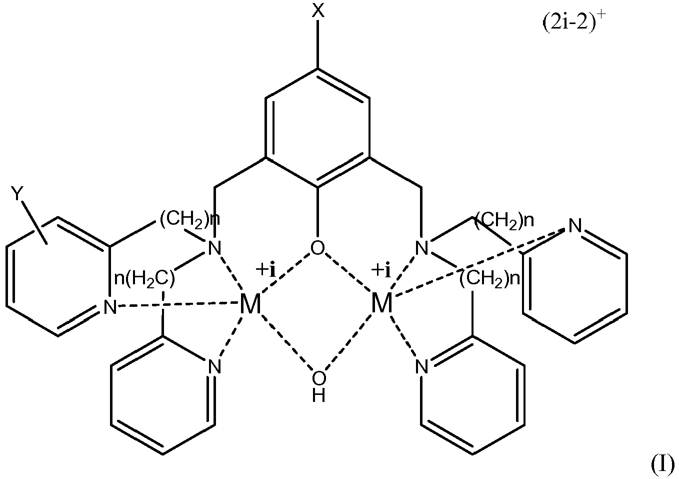 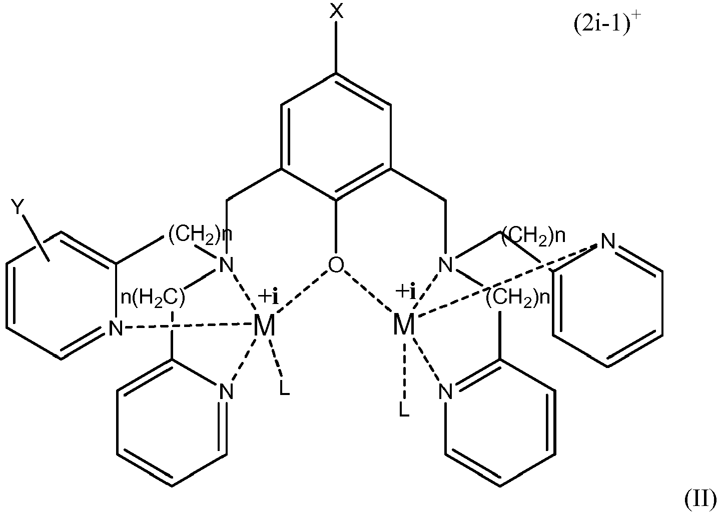 dans laquelle
- M⁺ⁱ représente un ion métallique et i est 1, 2 ou 3
- L représente un ligand échangeable constitué d'une molécule qui interagit de manière faible avec les ions métalliques et qui est disponible pour l'échange avec la solution environnante,
- X représente un groupe -(CH₂)ₘ-NH-A, ou un groupe -CH₂-NHC(O)-R-NH-A dans lequel R est un groupe alkyle en C₂-C₁₀, de préférence en C₆-C₁₀, linéaire ou ramifié et A est H ou un groupe fluorophore
- m = 1 à 12
- n = 1, 2 ou 3
- Y représente (CH₂)ₚ-NH₂ où p = 0 à 12.

2. Composé de formule (I) ou (II) selon la revendication 1, **caractérisé en ce que** M est choisi parmi Zn, Cu, Mn, Co, Ni et Fe.

3. Composé selon l'une des revendications 1 ou 2 de formule (I) ou (II) dans laquelle :
- M représente Zn ou Cu
- X représente -(CH₂)ₘ-NH-A où m= 1, A représente H, n=1 et p=0 ; ou
- X représente -(CH₂)ₘ-NH-A où m= 1, A représente H, n=2 et p=0 , ou
- X représente-CH₂-NHC(O)-R- NH-A, A représente H, n=1 et p=0.

4. Composé de formule (I) ou (II) selon l'une des revendications 1 ou 2, dans laquelle X représente -(CH₂)ₘ-NH-A, -CH₂-NHC(O)-R-NH-A où R est un groupe alkyle en C₂-C₁₀, de préférence en C₆-C₁₀, linéaire ou ramifié et A est un groupe fluorophore

5. Composé de formule (III) 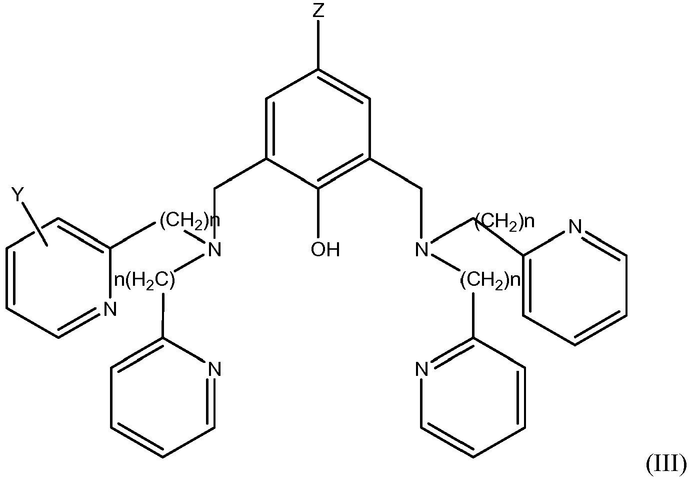 dans laquelle
- Z représente NH₂ ou un groupe -(CH₂)ₘ-NH-A, ou un groupe -CH₂-NHC(O)-R-NH-A dans lequel R est un groupe alkyle en C₂-C₁₀, de préférence en C₆-C₁₀, linéaire ou ramifié et A est H ou un groupe fluorophore, sous réserve que, lorsque m= 2, A soit différent de H
- m = 1 à 12
- n = 1, 2 ou 3
- Y représente (CH₂)ₚ-NH₂ où p = 0 à 12.

6. Composé de formule (III) selon la revendication 5, dans laquelle
- Z représente -(CH₂)ₘ-NH-A où m= 1, A représente H, n=1 et p=0 ; ou
- Z représente -(CH₂)ₘ-NH-A où m= 1, A représente H, n=2 et p=0 ; ou
- Z représente-CH₂-NHC(O)-R-NH-A, A représente H, n=1 et p=0.

7. Méthode de détection et/ou de caractérisation de microparticules cellulaires, **caractérisée en ce qu'**elle comprend la mise en contact d'un composé de formule (I) ou (II) 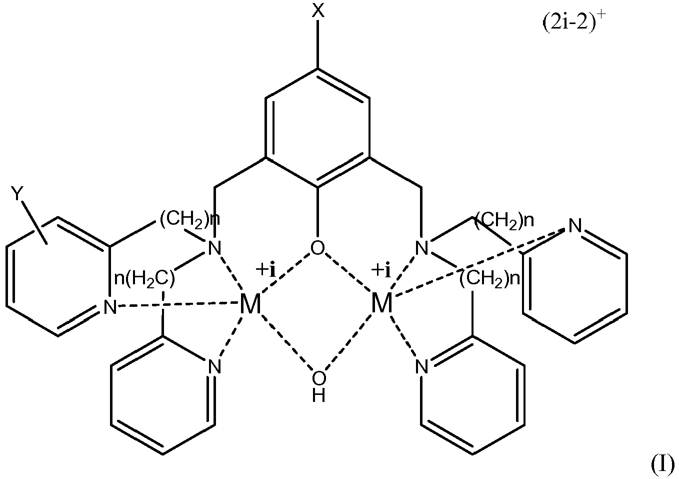 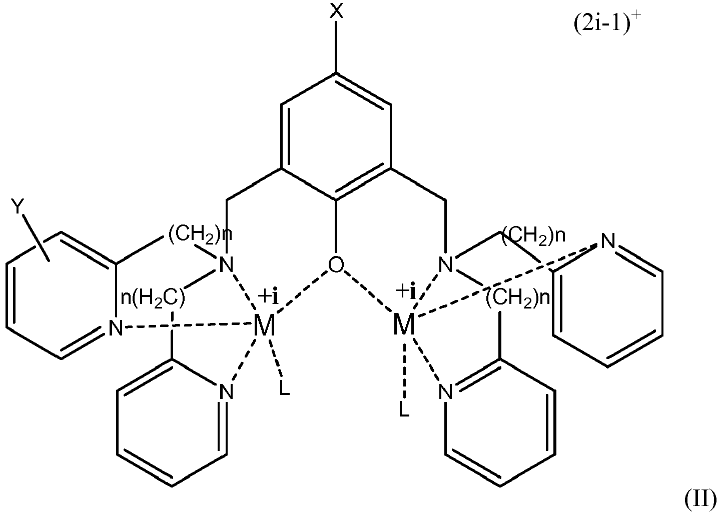 telle que définie dans l'une quelconque des revendications 1 à 4 avec un échantillon de fluide biologique susceptible de contenir des microparticules cellulaires et la détection et/ou la caractérisation des microparticules capturées par ledit composé de formule (I) ou (II).

8. Méthode selon la revendication 7, **caractérisée en ce que** le composé de formule (I) est, dans une première étape, immobilisé sur un support solide.

9. Méthode selon la revendication 8, **caractérisée en ce qu'**elle comprend les étapes suivantes :
- immobilisation d'un composé de formule (I) ou (II) sur la surface du support solide,
- mise en contact avec un échantillon de fluide biologique susceptible de contenir des microparticules cellulaires
- capture des microparticules cellulaires sur ledit composé de formule (I) ou (II), et
- détection et/ou caractérisation des microparticules cellulaires capturées.

10. Méthode selon les revendications 7 ou 8, **caractérisée en ce qu'**elle comprend les étapes suivantes :
- activation d'un support solide,
- immobilisation d'un composé de formule (I) ou (II) telle que définies ci-dessus sur la surface du support activé,
- mise en contact avec un échantillon de fluide biologique susceptible de contenir des microparticules cellulaires
- capture des microparticules cellulaires sur ledit composé de formule (I) ou (II), et
- détection et/ou caractérisation des microparticules cellulaires capturées.

11. Méthode selon la revendication 7, **caractérisée en ce qu'**elle comprend les étapes suivantes
- immobilisation d'un composé de formule (III) 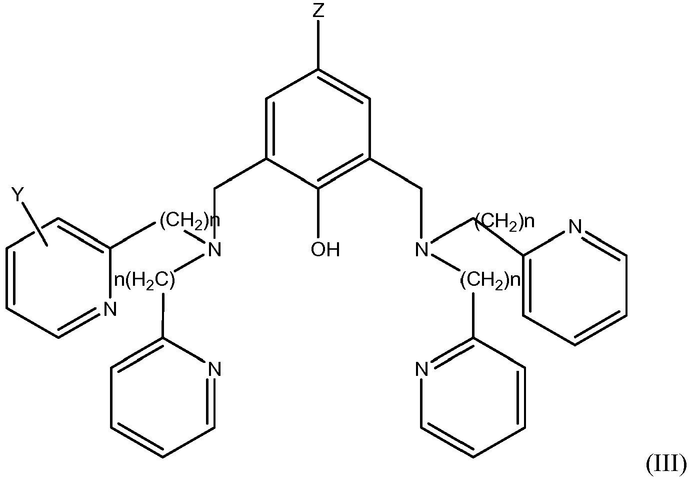 dans laquelle
- Z représente NH₂ ou un groupe -(CH₂)ₘ-NH-A, ou un groupe -CH₂-NHC(O)-R-NH-A dans lequel R est un groupe alkyle en C₂-C₁₀, de préférence en C₆-C₁₀, linéaire ou ramifié et A est H ou un groupe fluorophore, sous réserve que, lorsque m= 2, A soit différent de H
- m = 1 à 12
- n = 1, 2 ou 3
- Y représente (CH₂)ₚ-NH₂ où p = 0 à 12
sur un support solide
- incubation de l'ensemble support-ligand en présence d'ion métallique, de manière à ce que le complexe dinucléaire métallique de formule (I) ou (II) se forme *in situ*
- mise en contact dudit composé de formule (I) ou (II) avec un échantillon de fluide biologique susceptible de contenir des microparticules cellulaires,
- capture des microparticules cellulaires sur ledit composé de formule (I) ou (II), et
- détection et/ou caractérisation des microparticules cellulaires capturées.

12. Méthode selon la revendication 7, **caractérisée en ce que** le composé de formule (I) ou (II) est utilisé en solution.

13. Méthode selon la revendication 12, **caractérisée en ce qu'**on utilise un composé de formule (I) ou (II) dans laquelle X représente -(CH₂)ₘ-NH-A, -CH₂-NHC(O)-R-NH-A où R est un groupe alkyle en C₂-C₁₀, de préférence en C₆-C₁₀ linéaire ou ramifié et A est un groupe fluorophore

14. Méthode de détection et/ou de caractérisation de microparticules cellulaires selon l'une quelconque des revendications 7 à 13, permettant l'évaluation du risque de développement et/ou le suivi thérapeutique de diverses pathologies, qui comprend les étapes suivantes :
- la mise en contact d'un composé de formule (I) ou (II) telle que définie dans la revendication 7 avec un échantillon de fluide biologique susceptible de contenir des microparticules cellulaires,
- la détection et/ou la caractérisation des microparticules capturées par ledit composé de formule (I), et
- la comparaison du résultat de la mesure effectuée à l'étape précédente avec le résultat d'une mesure identique réalisée sur un échantillon de fluide biologique témoin.

15. Méthode de diagnostic, d'évaluation du risque de développement et/ou de suivi thérapeutique de diverses pathologies, qui comprend les étapes suivantes :
- la mise en contact d'un composé de formule (I) ou (II) telle que définie dans la revendication 7 avec un échantillon de fluide biologique susceptible de contenir des microparticules cellulaires,
- la détection et/ou la caractérisation des microparticules capturées par ledit composé de formule (I), et
- la comparaison du résultat de la mesure effectuée à l'étape précédente avec le résultat d'une mesure identique réalisée sur un échantillon de fluide biologique témoin.

16. Méthode selon l'une des revendications 14 ou 15, **caractérisée en ce que** soit le fluide biologique témoin est un fluide biologique identique à celui testé, mais provenant d'un individu considéré comme sain, soit le fluide biologique témoin provient du même individu que le fluide biologique testé, mais résulte d'un prélèvement antérieur.

17. Méthode selon l'une quelconque des revendications 14 à 16, **caractérisée en ce que** ladite pathologie est choisie parmi les pathologies thrombotiques, inflammatoires et/ou métaboliques, les maladies ou accidents cardiovasculaires ou neurovasculaires, le diabète, le cancer ou la maladie d'Alzheimer.

18. Méthode selon l'une quelconque des revendications 7 à 17, **caractérisée en ce que** les microparticules capturées sont révélées en utilisant des anticorps marqués spécifiques qui peuvent être détectés en utilisant une analyse photométrique ou mesure de fluorescence.

## Patentansprüche

1. Verbindung der Formel (I) oder der Formel (II): 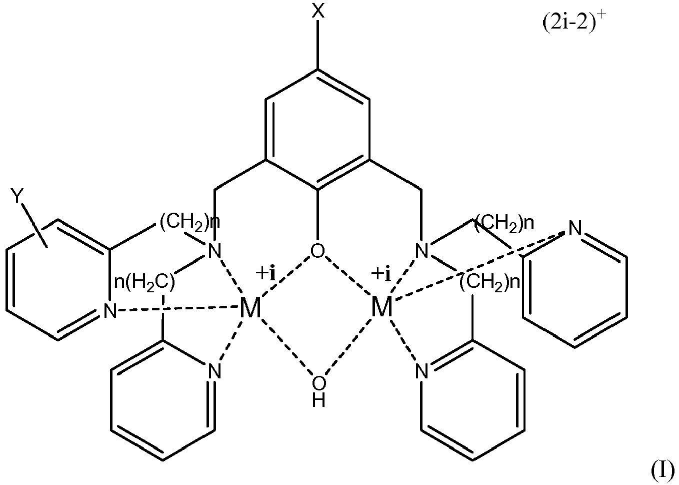 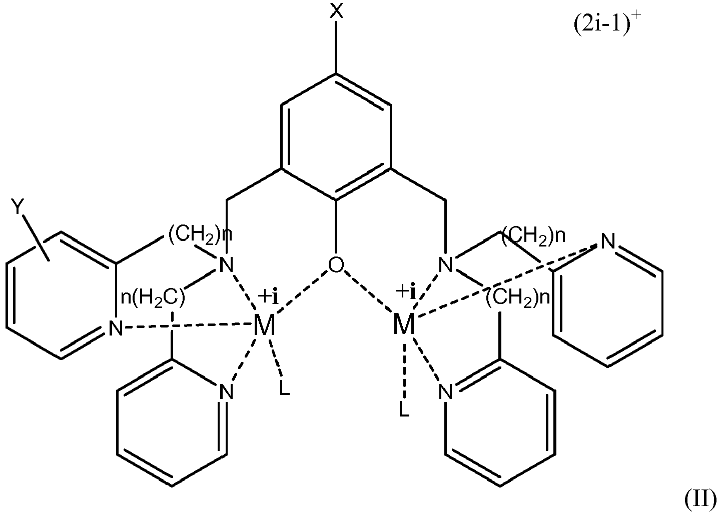 worin
- M⁺ⁱ ein Metallion darstellt und i 1, 2 oder 3 ist,
- L einen austauschbaren Liganden darstellt, der aus einem Molekül besteht, das schwach mit den Metallionen wechselwirkt und für den Austausch mit der umgebenden Lösung bereit steht,
- X eine -(CH₂)ₘ-NH-A-Gruppe oder eine -CH₂-NHC(O)-R-NH-A-Gruppe darstellt, wobei R eine lineare oder verzweigte C₂-C₁₀-, vorzugsweise C₆-C₁₀ - Alkylgruppe ist, und A H oder eine Fluorophor-Gruppe ist,
- m = 1 bis 12
- n = 1, 2 oder 3
- Y (CH₂)ₚ-NH₂ darstellt, worin p = 0 bis 12,
- wenn p = 0, die Pyridin-Gruppe nicht mit Y substituiert ist.

2. Verbindung der Formel (I) oder (II) nach Anspruch 1, **dadurch gekennzeichnet, dass** M ausgewählt ist aus Zn, Cu, Mn, Co, Ni und Fe.

3. Verbindung der Formel (I) oder (II) nach einem der Ansprüche 1 oder 2, wobei :
- M Zn oder Cu darstellt,
- X -(CH₂)ₘ-NH-A darstellt, worin m = 1, A H darstellt, n=1 und p=0; oder
- X -(CH₂)ₘ-NH-A darstellt, worin m= 1, A H darstellt, n=2 und p=0 , oder
- X -CH₂-NHC(O)-R- NH-A darstellt, A H darstellt, n=1 und p=0.

4. Verbindung der Formel (I) oder (II) nach einem der Ansprüche 1 oder 2, wobei X -(CH₂)ₘ-NH-A, -CH₂-NHC(O)-R-NH-A darstellt, worin R eine lineare oder verzweigte C₂-C₁₀-, vorzugsweise C₆-C₁₀- Alkylgruppe ist und A eine Fluorophor-Gruppe ist.

5. Verbindung der Formel (III) 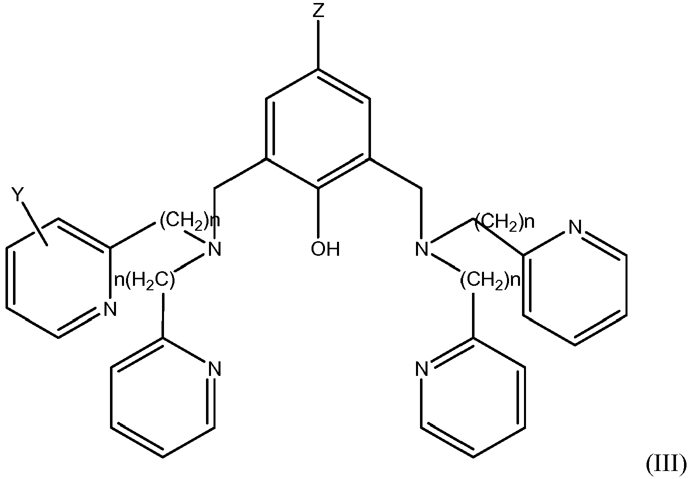 wobei
- Z NH₂ oder eine-(CH₂)ₘ-NH-A-Gruppe oder eine -CH₂-NHC(O)-R-NH-A-Gruppe darstellt, wobei R eine lineare oder verzweigte C₂-C₁₀-, vorzugsweise C₆-C₁₀- Alkylgruppe ist und A H oder eine Fluorophor-Gruppe ist, sofern dann, wenn m= 2, A verschieden von H ist,
- m = 1 bis 12
- n = 1, 2 oder 3
- Y (CH₂)ₚ-NH₂ darstellt, worin p = 0 bis 12.

6. Verbindung der Formel (III) nach Anspruch 5, wobei
- Z -(CH₂)ₘ-NH-A darstellt, worin m= 1, A H darstellt, n=1 und p=0; oder
- Z -(CH₂)ₘ-NH-A darstellt, worin m= 1, A H darstellt, n=2 und p=0; oder
- Z -CH₂-NHC(O)-R-NH-A darstellt, A H darstellt, n=1 und p=0.

7. Verfahren zum Nachweisen und/oder Charakterisieren von zellulären Mikropartikeln, **dadurch gekennzeichnet, dass** es das Inkontaktbringen einer Verbindung der Formel (I) oder (II) 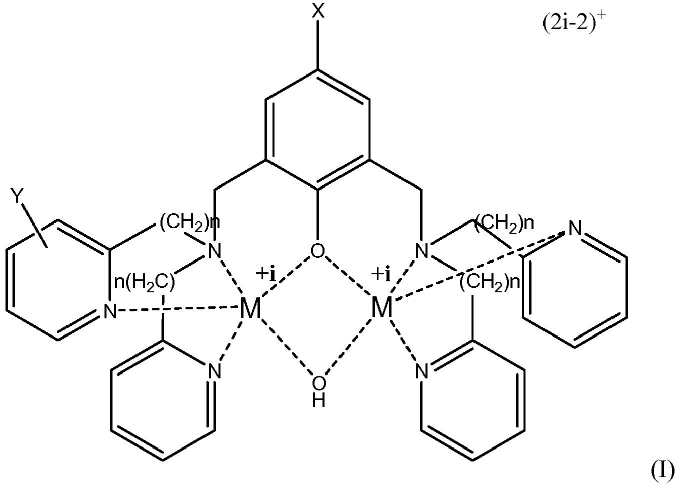 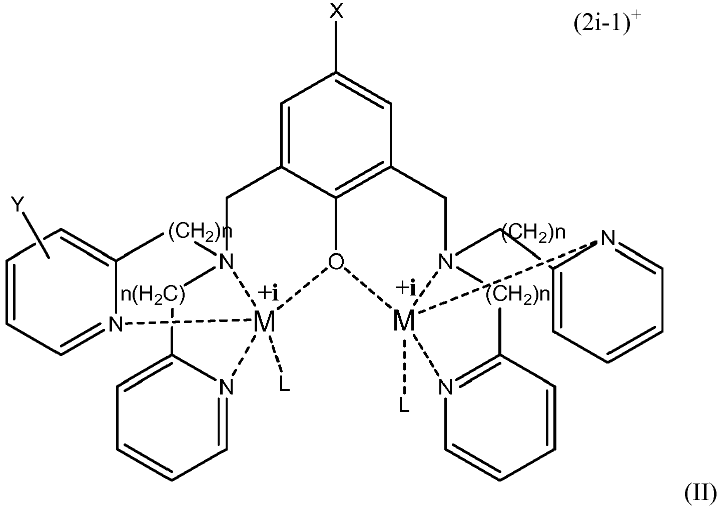 wie nach einem der Ansprüche 1 bis 4 definiert, mit einer biologischen Flüssigkeitsprobe umfasst, die zelluläre Mikropartikel enthalten kann, sowie das Nachweisen und/oder Charakterisieren von Mikropartikeln, die von der Verbindung der Formel (I) oder (II) abgefangen sind.

8. Verfahren nach Anspruch 7, **dadurch gekennzeichnet, dass** die Verbindung der Formel (I) in einem ersten Schritt auf einem festen Träger immobilisiert wird.

9. Verfahren nach Anspruch 8, **dadurch gekennzeichnet, dass** es die nachfolgenden Schritte umfasst:
- Immobilisieren einer Verbindung der Formel (I) oder (II) an der Oberfläche des festen Trägers,
- Inkontaktbringen mit einer biologischen Flüssigkeitsprobe, die zelluläre Mikropartikel enthalten kann,
- Abfangen der zellulären Mikropartikel an der Verbindung der Formel (I) oder (II), und
- Nachweisen und/oder Charakterisieren der abgefangenen zellulären Mikropartikel.

10. Verfahren nach den Ansprüchen 7 oder 8, **dadurch gekennzeichnet, dass** es die nachfolgenden Schritte umfasst:
- Aktivieren eines festen Trägers,
- Immobilisieren einer Verbindung der Formel (I) oder (II), wie oben definiert, an der Oberfläche des aktivierten Trägers,
- Inkontaktbringen mit einer biologischen Flüssigkeitsprobe, die zelluläre Mikropartikel enthalten kann,
- Abfangen der zellulären Mikropartikel an der Verbindung der Formel (I) oder (II), und
- Nachweisen und/oder Charakterisieren der abgefangenen zellulären Mikropartikel.

11. Verfahren nach Anspruch 7, **dadurch gekennzeichnet, dass** es die nachfolgenden Schritte umfasst
- Immobilisierung einer Verbindung der Formel (III) 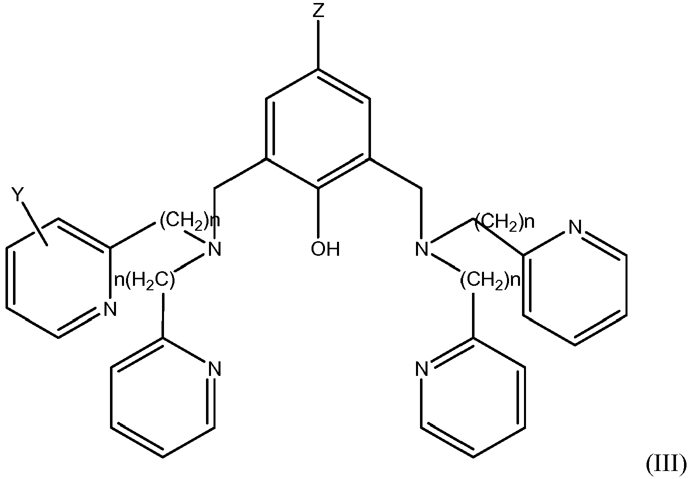 wobei
- Z NH₂ oder eine -(CH₂)ₘ-NH-A-Gruppe oder eine -CH₂-NHC(O)-R-NH-A- Gruppe darstellt, wobei R eine lineare oder verzweigte C₂-C₁₀-, vorzugsweise C₆-C₁₀ - Alkylgruppe ist und A H oder eine Fluorophor-Gruppe ist, sofern dann, wenn m= 2, A verschieden von H ist,
- m = 1 bis 12
- n = 1, 2 oder 3
- Y (CH₂)ₚ-NH₂ darstellt, worin p = 0 bis 12,
auf einen festen Träger,
- Inkubation der Träger-Ligand-Anordnung unter Gegenwart eines Metallions, so dass der dinukleare Metallkomplex der Formel (I) oder (II) *in situ* entsteht,
- Inkontaktbringen der Verbindung der Formel (I) oder (II) mit einer biologischen Flüssigkeitsprobe, die zelluläre Mikropartikel enthalten kann,
- Abfangen der zellulären Mikropartikel an der Verbindung der Formel (I) oder (II), und
- Nachweisen und/oder Charakterisieren der abgefangenen zellulären Mikropartikel.

12. Verfahren nach Anspruch 7, **dadurch gekennzeichnet, dass** die Verbindung der Formel (I) oder (II) in Lösung verwendet wird.

13. Verfahren nach Anspruch 12, **dadurch gekennzeichnet, dass** eine Verbindung der Formel (I) oder (II) verwendet wird, wobei X -(CH₂)ₘ-NH-A, -CH₂-NHC(O)-R-NH-A darstellt, worin R eine lineare oder verzweigte C₂-C₁₀-, vorzugsweise C₆-C₁₀ - Alkylgruppe ist und A eine Fluorophor-Gruppe ist.

14. Verfahren zum Nachweisen und/oder Charakterisieren von zellulären Mikropartikeln nach einem der Ansprüche 7 bis 13, mit dem die Bewertung des Entwicklungsrisikos und/oder die therapeutische Behandlung verschiedener Erkrankungen möglich ist, das nachfolgende Schritte umfasst:
- Inkontaktbringen einer Verbindung der Formel (I) oder (II), wie in Anspruch 7 definiert, mit einer biologischen Flüssigkeitsprobe, die zelluläre Mikropartikel enthalten kann,
- Nachweisen und/oder Charakterisieren der mit der Verbindung der Formel (I) abgefangenen Mikropartikel, und
- Vergleichen des Ergebnisses der im vorherigen Schritt erfolgten Messung mit dem Ergebnis einer identischen Messung, die an einer biologischen Kontrollflüssigkeitsprobe durchgeführt wurde.

15. Verfahren zur Diagnose, Bewertung des Entwicklungsrisikos und/oder zur therapeutischen Behandlung verschiedener Erkrankungen, das nachfolgende Schritte umfasst:
- Inkontaktbringen einer Verbindung der Formel (I) oder (II), wie in Anspruch 7 definiert, mit einer biologischen Flüssigkeitsprobe, die zelluläre Mikropartikel enthalten kann,
- Nachweisen und/oder Charakterisieren der mit der Verbindung der Formel (I) abgefangenen Mikropartikel, und
- Vergleichen des Ergebnisses der im vorherigen Schritt erfolgten Messung mit dem Ergebnis einer identischen Messung, die an einer biologischen Kontrollflüssigkeitsprobe durchgeführt wurde.

16. Verfahren nach einem der Ansprüche 14 oder 15, **dadurch gekennzeichnet, dass** entweder die biologische Kontrollflüssigkeit eine biologische Flüssigkeit identisch zur getesteten ist, jedoch von einer als gesund erachteten Person stammt, oder die biologische Kontrollflüssigkeit von der gleichen Person wie die getestete biologische Flüssigkeit stammt, jedoch von einer früheren Entnahme resultiert.

17. Verfahren nach einem der Ansprüche 14 bis 16, **dadurch gekennzeichnet, dass** die Erkrankung ausgewählt ist aus thrombotischen, entzündlichen und/oder metabolischen Erkrankungen, kardiovaskulären oder neurovaskulären Erkrankungen oder Unfällen, Diabetes, Krebs oder Alzheimer-Krankheit.

18. Verfahren nach einem der Ansprüche 7 bis 17, **dadurch gekennzeichnet, dass** die abgefangenen Mikropartikel unter Verwendung spezifischer markierter Antikörper erkannt werden, die unter Verwendung einer photometrischen Analyse oder Fluoreszenzmessung nachgewiesen werden können.

## Claims

1. A compound having formula (I) or formula (II): 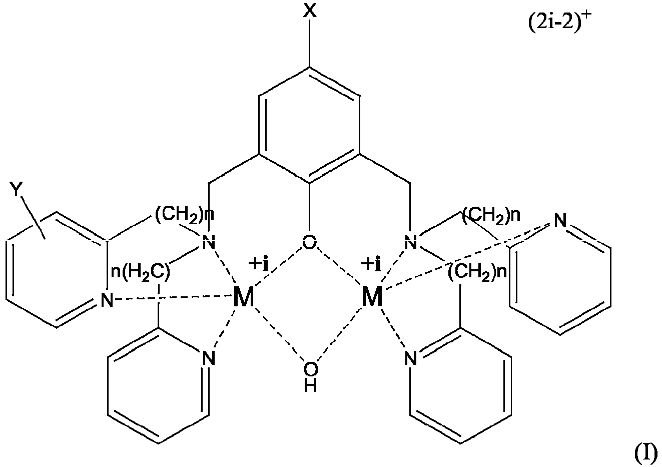 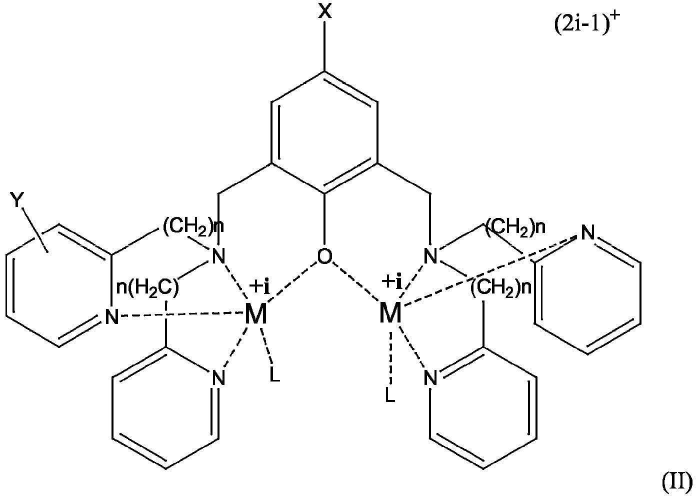 wherein
- M⁺ⁱ is a metal ion, and i is 1, 2 or 3,
- L is an exchangeable ligand consisting of a molecule which interacts weakly with the metal ions and which is available for exchange with the surrounding solution,
- X is a -(CH₂)ₘ-NH-A group, or a -CH₂-NHC(O)-R-NH-A group wherein R is a C₂-C₁₀ alkyl group, preferably a C₆-C₁₀ alkyl group, either linear or branched, and A is H or a fluorophore group,
- m = 1 to 12,
- n = 1, 2 or 3,
- Y is (CH₂)ₚ-NH₂ where p = 0 to 12,
- when p = 0, the pyridine group is not substituted by Y.

2. A compound having formula (I) or formula (II) according to claim 1, **characterised in that** M is selected from Zn, Cu, Mn, Co, Ni and Fe.

3. A compound according to one of claims 1 or 2 having formula (I) or (II) wherein:
- M is Zn or Cu
- X is -(CH₂)ₘ-NH-A, where m = 1, A is H, n = 1 and p = 0; or
- X is -(CH₂)ₘ-NH-A, where m = 1, A is H, n = 2 and p = 0; or
- X is -CH₂-NHC(O)-R-NH-A, A is H, n = 1 and p = 0.

4. A compound having formula (I) or formula (II) according to one of claims 1 or 2, wherein X is -(CH₂)ₘ-NH-A, -CH₂-NHC(O)-R-NH-A where R is a C₂-C₁₀ alkyl group, preferably a C₆-C₁₀ alkyl group, either linear or branched, and A is a fluorophore group.

5. A compound having formula (III) 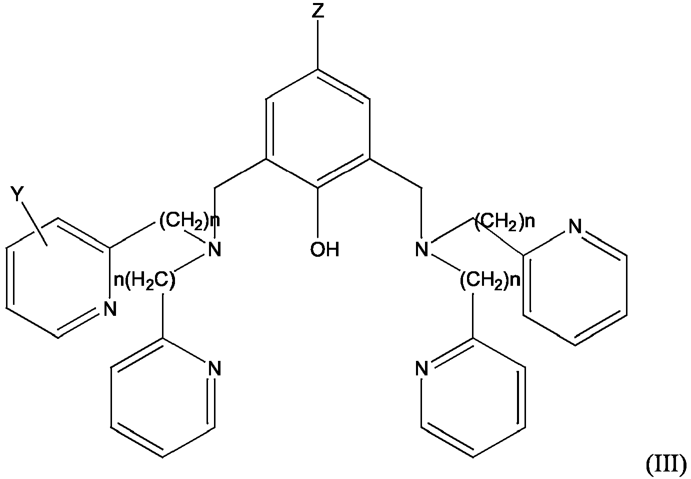 wherein
- Z is NH₂ or a -(CH₂)ₘ-NH-A group, or a -CH₂-NHC(O)-R-NH-A group wherein R is a C₂-C₁₀ alkyl group, preferably a C₆-C₁₀ alkyl group, either linear or branched, and A is H or a fluorophore group, provided that when m = 2, A is different from H,
- m = 1 to 12,
- n = 1, 2 or 3,
- Y is (CH₂)ₚ-NH₂ where p = 0 to 12.

6. A compound having formula (III) according to claim 5, wherein
- Z is -(CH₂)ₘ-NH-A, where m = 1, A is H, n = 1 and p = 0; or
- Z is -(CH₂)ₘ-NH-A, where m = 1, A is H, n = 2 and p = 0; or
- Z is -CH₂-NHC(O)-R-NH-A, A is H, n = 1 and p = 0.

7. A method for detection and/or characterisation of cellular microparticles, **characterised in that** it comprises the placing in contact of a compound having formula (I) or (II) 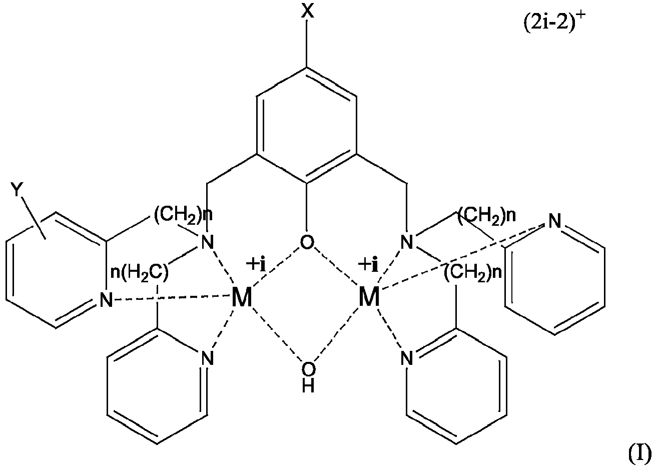 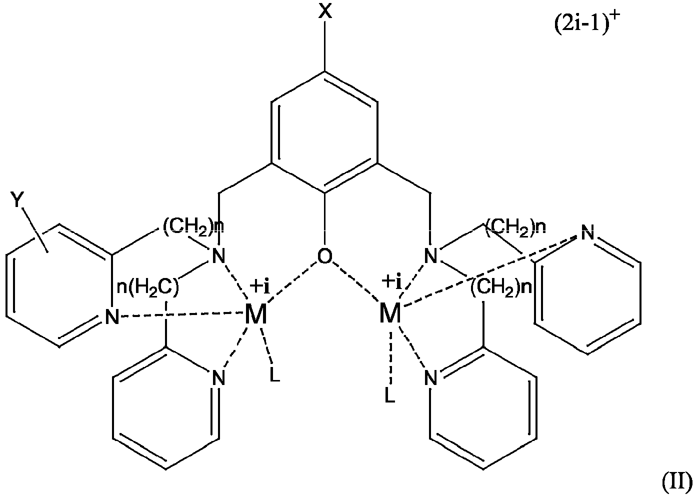 as defined in any one of claims 1 to 4 with a sample of biological fluid that is likely to contain cellular microparticles, and the detection and/or characterisation of the microparticles captured by said compound having formula (I) or (II).

8. A method according to claim 7, **characterised in that** the compound having formula (I) is, in a first step, immobilised on a solid support.

9. A method according to claim 8, **characterised in that** it comprises the following steps:
- immobilisation of a compound having formula (I) or (II) on the surface of the solid support,
- placing in contact with a sample of biological fluid that is likely to contain cellular microparticles,
- capture of the cellular microparticles on said compound having formula (I) or (II), and
- detection and/or characterisation of the captured cellular microparticles.

10. A method according to claims 7 or 8, **characterised in that** it comprises the following steps:
- activation of a solid support,
- immobilisation of a compound having formula (I) or (II) as defined here above on the surface of the activated support,
- placing in contact with a sample of biological fluid that is likely to contain cellular microparticles,
- capture of the cellular microparticles on said compound having formula (I) or (II), and
- detection and/or characterisation of the captured cellular microparticles.

11. A method according to claim 7, **characterised in that** it comprises the following steps:
- immobilisation of a compound having formula (III) 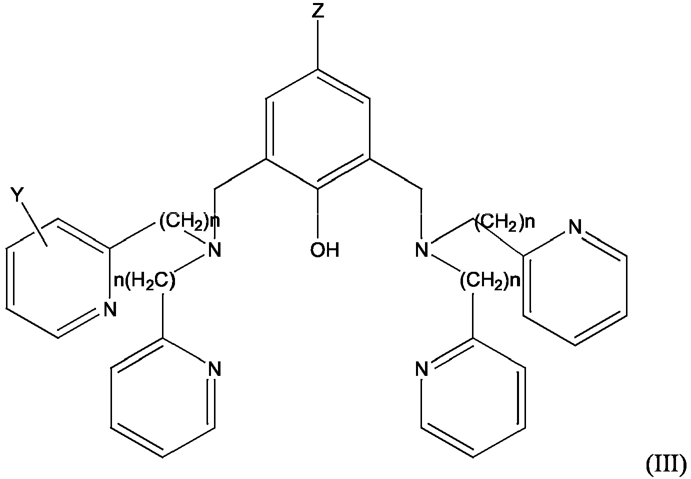 wherein
- Z is NH₂ or a -(CH₂)ₘ-NH-A group, or a -CH₂-NHC(O)-R-NH-A group wherein R is a C₂-C₁₀ alkyl group, preferably a C₆-C₁₀ alkyl group, either linear or branched, and A is H or a fluorophore group, provided that when m = 2, A is different from H,
- m = 1 to 12,
- n = 1, 2 or 3,
- Y is (CH₂)ₚ-NH₂ where p = 0 to 12
on a solid support
- incubation of the support-ligand ensemble in the presence of the metal ion, in a manner such that the dinuclear metal complex having formula (I) or (II) is formed *in situ*
- the placing in contact of said compound having formula (I) or (II) with a sample of biological fluid that is likely to contain cellular microparticles,
- capture of the cellular microparticles on said compound having formula (I) or (II), and
- detection and/or characterisation of the captured cellular microparticles.

12. A method according to claim 7, **characterised in that** the compound having formula (I) or (II) is used in a solution.

13. A method according to claim 12, **characterised in that** use is made of a compound having formula (I) or (II) wherein X is -(CH₂)ₘ-NH-A, -CH₂-NHC(O)-R-NH-A where R is a C₂-C₁₀ alkyl group, preferably a C₆-C₁₀ alkyl group, either linear or branched, and A is a fluorophore group.

14. A method for detection and/or characterisation of cellular microparticles according to any one of claims 7 to 13, that enables the assessment of the risk of developing various diseases and/or the therapeutic follow up and monitoring thereof which comprises the following steps:
- the placing in contact of a compound having formula (I) or (II) as defined in claim 7 with a sample of biological fluid that is likely to contain cellular microparticles,
- the detection and/or characterisation of the captured microparticles by said compound having formula (I), and
- the comparison of the results of the measurement performed in the previous step with the results of a similar measurement performed on a control sample of biological fluid.

15. A method for diagnosis, and assessment of the risk of developing various diseases and/or the therapeutic follow up and monitoring thereof, which comprises the following steps:
- the placing in contact of a compound having formula (I) or (II) as defined in claim 7 with a sample of biological fluid that is likely to contain cellular microparticles,
- the detection and/or characterisation of the captured microparticles by said compound having formula (I), and
- the comparison of the results of the measurement performed in the previous step with the results of a similar measurement performed on a control sample of biological fluid.

16. A method according to one of claims 14 or 15, **characterised in that** either the control biological fluid is a biological fluid that is identical to that tested, but derived from an individual considered to be healthy, or the control biological fluid is from the same individual as the biological fluid tested, but is derived from an earlier sample.

17. A method according any one of claims 14 to 16, **characterised in that** said pathology is selected from thrombotic, inflammatory and/or metabolic disorders, cardiovascular or neurovascular diseases or events, diabetes, cancer, or Alzheimer's disease.

18. A method according to any one of claims 7 to 17, **characterised in that** the captured microparticles are revealed by using specific labelled antibodies that may be detected by means of photometric analysis or using fluorescence measurement.
